# EUROPEAN PATENT APPLICATION

(11) **EP 4 317 549 A1**
(43) Date of publication of application: **07.02.2024**
(21) Application number: 22779050.8
(22) Date of filing: 30.03.2022
(51) Int. Cl.: C40B 50/06, C40B 40/02

(54) **VECTOR AND METHOD FOR SCREENING FUNCTIONAL ANTIGEN-BINDING PROTEIN**

(30) Priority: 31.03.2021 CN 202110350207
(71) Applicant: Ddbio. Co, Ltd., (Shang Hai), Pudong Shanghai 201210 (CN)
(72) Inventor: ZHOU, Chen, Shanghai 201210 (CN)
(74) Representative: Weickmann & Weickmann PartmbB
(86) International application number: PCT/CN2022/084229
(87) International publication number: WO 2022/206868

(57) **Abstract**

A method for selecting a functional antigen-binding protein. The method comprises the step of constructing an expression vector, by means of a phage display technique and/or a cell display technique, using a light chain or a fragment thereof of a reference antigen-binding protein with a known sequence and function, or a heavy chain or a fragment thereof of the reference antigen-binding protein, thereby selecting a functional antigen-binding protein that binds to the same epitope as the reference antigen-binding protein. Further provided is a functional antigen-binding protein obtained by means of the method.

## Description

### BACKGROUND OF THE INVENTION

### 1. Technical Field

The present application relates to the field of biomedicine, and in particular to vectors for constructing and expressing an antigen-binding protein, and a method for screening a functional antigen-binding protein.

### 2. Description of Related Art

At present, the widely used methods in the antibody industry for obtaining functional antibodies, or antigen-binding fragments thereof, with promising properties mainly include hybridoma technology and antibody library technology. However, the hybridoma technology has the problems of small number of candidate clones and easy loss of positive clones, and requires a lot of time and effort for the selection of positive clones, especially for the complex functional screening. The antibody library technology involves cloning, with genetic engineering methods, variable region genes of antibody heavy and light chains into plasmids or phages for expression, and then screening out clones carrying specific antibody genes by means of different antigens. It mainly includes a phage display technique and a yeast surface display technique. The antibody library technology greatly improves the library capacity. However, an antigen of interest generally has a plurality of (for example, several, dozens of, or even hundreds of) antigenic determinants, and there may be only one or several antigenic determinants with the desired biological function. When antigen-specific antibodies or antigen-binding fragments thereof are screened against a target of interest, it is not known which antigen determinant has the desired biological function. In the prior art, it is typical to first obtain a large number of antigen-specific antibodies or antigen-binding fragments thereof, and then perform analysis and identification one by one to find out the antibodies, or antigen-binding fragments thereof, that bind to specific antigenic determinants and have the desired biological function. In this way, a long time and a large amount of work are still needed, and the screening effect is poor. Therefore, there is a need of a new technique for discovering antibodies or antigen-binding fragments thereof, in order to improve the quality, quantity, and diversity of lead molecules, accelerate the drug development, and improve the development success ratio.

In one aspect, the present application provides a method for selecting a functional antigen-binding protein, including: a) providing a first polynucleotide including R1-nucleic acid fragment I-R2 in a 5'-to-3' direction, the nucleic acid fragment I being capable of encoding an antigen-binding fragment I; b) providing a second polynucleotide including R3-reference nucleic acid fragment II-R4 in a 5'-to-3' direction, the reference nucleic acid fragment II being capable of encoding a reference antigen-binding fragment II, which, together with a reference antigen-binding fragment I encoded by a reference nucleic acid fragment I, is capable of forming a reference antigen-binding protein; c) providing a first vector polynucleotide including R2-vector fragment I-R3 in a 5'-to-3' direction; d) providing a second vector polynucleotide including R4-vector fragment II-R1 in a 5'-to-3' direction; e) cleaving, with a restriction endonuclease, the first polynucleotide, the second polynucleotide, the first vector polynucleotide and the second vector polynucleotide to obtain the cleaved first polynucleotide, the cleaved second polynucleotide, the cleaved first vector polynucleotide and the cleaved second vector polynucleotide; f) mixing the cleaved first polynucleotide, the cleaved second polynucleotide, the cleaved first vector polynucleotide and the cleaved second vector polynucleotide, such that the cleaved first polynucleotide, the cleaved second polynucleotide, the cleaved first vector polynucleotide and the cleaved second vector polynucleotide can be cyclized by directional linkage to form first to-be-screened vectors; g) expressing the first to-be-screened vectors, and selecting a first to-be-screened vector capable of expressing an expression product having the following property, as a first replacement vector: capability of binding to a target, to which the reference antigen-binding protein can bind, at a capacity that is 30% or more of a binding capacity of the reference antigen-binding protein to the target; and h) deriving the functional antigen-binding protein from the first replacement vector, wherein R1, R2, R3 and R4 are each independently a restriction endonuclease recognition site.

In some embodiments, the method includes cleaving the first polynucleotide with a restriction endonuclease specifically recognizing R1 and R2, to obtain the cleaved first polynucleotide.

In some embodiments, the method includes cleaving the second polynucleotide with a restriction endonuclease specifically recognizing R3 and R4, to obtain the cleaved second polynucleotide.

In some embodiments, the method includes cleaving the first vector polynucleotide with a restriction endonuclease specifically recognizing R2 and R3, to obtain the cleaved first vector polynucleotide.

In some embodiments, the method includes cleaving the second vector polynucleotide with a restriction endonuclease specifically recognizing R4 and R1, to obtain the cleaved second vector polynucleotide.

In some embodiments, a terminus of R1 resulting from specific cleavage by the restriction endonuclease specifically recognizing R1 and a terminus of any one of R2, R3 and R4 resulting from specific cleavage by the corresponding restriction endonuclease specifically recognizing the any one of R2, R3 and R4 do not recognize or link to each other.

In some embodiments, a terminus of R2 resulting from specific cleavage by the restriction endonuclease specifically recognizing R2 and a terminus of any one of R1, R3 and R4 resulting from specific cleavage by the corresponding restriction endonuclease specifically recognizing the any one of R1, R3 and R4 do not recognize or link to each other.

In some embodiments, a terminus of R3 resulting from specific cleavage by the restriction endonuclease specifically recognizing R3 and a terminus of any one of R1, R2 and R4 resulting from specific cleavage by the corresponding restriction endonuclease specifically recognizing the any one of R1, R2 and R4 do not recognize or link to each other.

In some embodiments, a terminus of R4 resulting from specific cleavage by the restriction endonuclease specifically recognizing R4 and a terminus of any one of R1, R2 and R3 resulting from specific cleavage by the corresponding restriction endonuclease specifically recognizing the any one of R1, R2 and R3 do not recognize or link to each other.

In some embodiments, the restriction endonuclease is selected from the group consisting of SfiI and BsmBI.

In some embodiments, the method includes introducing the first to-be-screened vector into a cell to express the first to-be-screened vector.

In some embodiments, the method includes introducing the first to-be-screened vector into a bacterium to prepare a phage library including one or more of the first to-be-screened vectors, thereby obtaining the functional antigen-binding protein from the phage library.

In some embodiments, the vector fragment I includes a linker, and the vector fragment II is derived from a display vector.

In some embodiments, the nucleic acid fragment I encodes an antibody light chain or a fragment thereof; the vector fragment I includes the linker; the reference nucleic acid fragment II encodes an antibody heavy chain or a fragment thereof; and the vector fragment II is derived from the display vector.

In some embodiments, R1 includes a nucleotide sequence as set forth in SEQ ID NO: 1.

In some embodiments, R2 includes a nucleotide sequence as set forth in SEQ ID NO: 2.

In some embodiments, R3 includes a nucleotide sequence as set forth in SEQ ID NO: 3.

In some embodiments, R4 includes a nucleotide sequence as set forth in SEQ ID NO: 4.

In some embodiments, the vector fragment II includes a linker, and the vector fragment I is derived from a display vector.

In some embodiments, the nucleic acid fragment I encodes an antibody heavy chain or a fragment thereof; the vector fragment I is derived from the display vector; the reference nucleic acid fragment II encodes an antibody light chain or a fragment thereof; and the vector fragment II includes the linker.

In some embodiments, R1 includes a nucleotide sequence as set forth in SEQ ID NO: 3.

In some embodiments, R2 includes a nucleotide sequence as set forth in SEQ ID NO: 4.

In some embodiments, R3 includes a nucleotide sequence as set forth in SEQ ID NO: 1.

In some embodiments, R4 includes a nucleotide sequence as set forth in SEQ ID NO: 2.

In some embodiments, the display vector is derived from a pComb3x vector.

In some embodiments, the linker includes a nucleic acid sequence encoding a signal peptide pelB or a fragment thereof.

In some embodiments, the linker has a length of about 50 bases to about 200 bases.

In some embodiments, the method includes introducing the first to-be-screened vector into a bacterium to obtain a DNA of the first to-be-screened vector from the bacterium, and introducing the DNA of the first to-be-screened vector into a cell, from which a functional antigen-specific binding polypeptide is obtained.

In some embodiments, the cell is a mammalian cell.

In some embodiments, the vector fragment I and/or the vector fragment II are/is derived from a mammalian cell expression vector.

In some embodiments, the mammalian cell expression vector is derived from pDGB4.

In some embodiments, the nucleic acid fragment I encodes an antibody light chain or a fragment thereof; and the reference nucleic acid fragment II encodes an antibody heavy chain or a fragment thereof.

In some embodiments, R1 includes a nucleotide sequence as set forth in SEQ ID NO: 7.

In some embodiments, R2 includes a nucleotide sequence as set forth in SEQ ID NO: 8.

In some embodiments, R3 includes a nucleotide sequence as set forth in SEQ ID NO: 5.

In some embodiments, R4 includes a nucleotide sequence as set forth in SEQ ID NO: 6.

In some embodiments, the nucleic acid fragment I encodes an antibody heavy chain or a fragment thereof; and the reference nucleic acid fragment II encodes an antibody light chain or a fragment thereof.

In some embodiments, R1 includes a nucleotide sequence as set forth in SEQ ID NO: 5.

In some embodiments, R2 includes a nucleotide sequence as set forth in SEQ ID NO: 6.

In some embodiments, R3 includes a nucleotide sequence as set forth in SEQ ID NO: 7.

In some embodiments, R4 includes a nucleotide sequence as set forth in SEQ ID NO: 8.

In some embodiments, the method includes: a) providing a third polynucleotide including R5-nucleic acid fragment I'-R6 in a 5'-to-3' direction; b) providing a fourth polynucleotide including R7-nucleic acid fragment II'-R8 in a 5'-to-3' direction, wherein the nucleic acid fragment II' is capable of encoding an antigen-binding protein II', and an antigen-binding fragment II', together with the reference antigen-binding fragment I, is capable of forming an antigen-binding protein having the following property: capability of binding to a target, to which the reference antigen-binding protein can bind, at a capacity that is 30% or more of a binding capacity of the reference antigen-binding protein to the target; c) providing a third vector polynucleotide including R6-vector fragment III-R7 in a 5'-to-3' direction; d) providing a fourth vector polynucleotide including R8-vector fragment IV-R5 in a 5'-to-3' direction; e) cleaving, with a restriction endonuclease, the third polynucleotide, the fourth polynucleotide, the third vector polynucleotide and the fourth vector polynucleotide to obtain the cleaved third polynucleotide, the cleaved fourth polynucleotide, the cleaved third vector polynucleotide and the cleaved fourth vector polynucleotide; f) mixing the cleaved third polynucleotide, the cleaved fourth polynucleotide, the cleaved third vector polynucleotide and the cleaved fourth vector polynucleotide, such that the cleaved third polynucleotide, the cleaved fourth polynucleotide, the cleaved third vector polynucleotide and the cleaved fourth vector polynucleotide can be cyclized by directional linkage to form double-replacement to-be-screened vectors; g) expressing the double-replacement to-be-screened vectors, and selecting a double-replacement to-be-screened vector capable of expressing an expression product having the following property, as a double-replacement vector: capability of binding to a target at a capacity that is 30% or more of a binding capacity of the reference antigen-binding protein to the target; and h) deriving the functional antigen-binding protein from the double-replacement vector, wherein R5, R6, R7 and R8 are each independently the restriction endonuclease recognition site.

In some embodiments, the method includes cleaving the third polynucleotide with a restriction endonuclease specifically recognizing R5 and R6, to obtain the cleaved third polynucleotide.

In some embodiments, the method includes cleaving the fourth polynucleotide with a restriction endonuclease specifically recognizing R7 and R8, to obtain the cleaved fourth polynucleotide.

In some embodiments, the method includes cleaving the third vector polynucleotide with a restriction endonuclease specifically recognizing R6 and R7, to obtain the cleaved third vector polynucleotide.

In some embodiments, the method includes cleaving the fourth vector polynucleotide with a restriction endonuclease specifically recognizing R8 and R5, to obtain the cleaved fourth vector polynucleotide.

In some embodiments, a terminus of R5 resulting from specific cleavage by the restriction endonuclease specifically recognizing R5 and a terminus of any one of R6, R7 and R8 resulting from specific cleavage by the corresponding restriction endonuclease specifically recognizing the any one of R6, R7 and R8 do not recognize or link to each other.

In some embodiments, a terminus of R6 resulting from specific cleavage by the restriction endonuclease specifically recognizing R6 and a terminus of any one of R5, R7 and R8 resulting from specific cleavage by the corresponding restriction endonuclease specifically recognizing the any one of R5, R7 and R8 do not recognize or link to each other.

In some embodiments, a terminus of R7 resulting from specific cleavage by the restriction endonuclease specifically recognizing R7 and a terminus of any one of R5, R6 and R8 resulting from specific cleavage by the corresponding restriction endonuclease specifically recognizing the any one of R5, R6 and R8 do not recognize or link to each other.

In some embodiments, a terminus of R8 resulting from specific cleavage by the restriction endonuclease specifically recognizing R8 and a terminus of any one of R5, R6 and R7 resulting from specific cleavage by the corresponding restriction endonuclease specifically recognizing the any one of R5, R6 and R7 do not recognize or link to each other.

In some embodiments, the restriction endonuclease is selected from the group consisting of SfiI and BsmBI.

In some embodiments, the method includes introducing the double-replacement to-be-screened vector into a cell to express the double-replacement to-be-screened vector.

In some embodiments, the method includes introducing the double-replacement to-be-screened vector into a bacterium to prepare a phage library including one or more of the double-replacement to-be-screened vectors, thereby obtaining the functional antigen-binding protein from the phage library.

In some embodiments, the vector fragment III includes a linker, and the vector fragment IV is derived from a display vector.

In some embodiments, the nucleic acid fragment I' encodes an antibody light chain or a fragment thereof; the vector fragment III includes the linker; the nucleic acid fragment II' encodes an antibody heavy chain or a fragment thereof; and the vector fragment IV is derived from the display vector.

In some embodiments, R5 includes a nucleotide sequence as set forth in SEQ ID NO: 1.

In some embodiments, R6 includes a nucleotide sequence as set forth in SEQ ID NO: 2.

In some embodiments, R7 includes a nucleotide sequence as set forth in SEQ ID NO: 3.

In some embodiments, R8 includes a nucleotide sequence as set forth in SEQ ID NO: 4.

In some embodiments, the vector fragment IV includes a linker, and the vector fragment III is derived from a display vector.

In some embodiments, the nucleic acid fragment I' encodes an antibody heavy chain or a fragment thereof; the vector fragment III is derived from the display vector; the nucleic acid fragment II' encodes an antibody light chain or a fragment thereof; and the vector fragment IV includes the linker.

In some embodiments, R5 includes a nucleotide sequence as set forth in SEQ ID NO: 3.

In some embodiments, R6 includes a nucleotide sequence as set forth in SEQ ID NO: 4.

In some embodiments, R7 includes a nucleotide sequence as set forth in SEQ ID NO: 1.

In some embodiments, R8 includes a nucleotide sequence as set forth in SEQ ID NO: 2.

In some embodiments, the display vector is derived from a pComb3x vector.

In some embodiments, the linker includes a nucleic acid sequence encoding a signal peptide pelB or a fragment thereof.

In some embodiments, the linker has a length of about 50 bases to about 200 bases.

In some embodiments, the method includes introducing the double-display to-be-screened vector into a bacterium to obtain a DNA of the double-display to-be-screened vector from the bacterium, and introducing the DNA of the double-display to-be-screened vector into a cell, from which a functional antigen-specific binding polypeptide is obtained.

In some embodiments, the cell is a mammalian cell.

In some embodiments, the vector fragment III and/or the vector fragment IV are/is derived from a mammalian expression vector.

In some embodiments, the mammalian expression vector is derived from pDGB4.

In some embodiments, the nucleic acid fragment I' encodes an antibody light chain or a fragment thereof; and the nucleic acid fragment II' encodes an antibody heavy chain or a fragment thereof.

In some embodiments, R5 includes a nucleotide sequence as set forth in SEQ ID NO: 7.

In some embodiments, R6 includes a nucleotide sequence as set forth in SEQ ID NO: 8.

In some embodiments, R7 includes a nucleotide sequence as set forth in SEQ ID NO: 5.

In some embodiments, R8 includes a nucleotide sequence as set forth in SEQ ID NO: 6.

In some embodiments, the nucleic acid fragment I' encodes an antibody heavy chain or a fragment thereof; and the nucleic acid fragment II' encodes an antibody light chain or a fragment thereof.

In some embodiments, R5 includes a nucleotide sequence as set forth in SEQ ID NO: 5.

In some embodiments, R6 includes a nucleotide sequence as set forth in SEQ ID NO: 6.

In some embodiments, R7 includes a nucleotide sequence as set forth in SEQ ID NO: 7.

In some embodiments, R8 includes a nucleotide sequence as set forth in SEQ ID NO: 8.

In some embodiments, the method includes: a) providing a fifth polynucleotide including R9-reference nucleic acid fragment I-R10 in a 5'-to-3' direction; b) providing a sixth polynucleotide including R11-nucleic acid fragment II-R12 in a 5'-to-3' direction; c) providing a fifth vector polynucleotide including R10-vector fragment V-R11 in a 5'-to-3' direction; d) providing a sixth vector polynucleotide including R12-vector fragment VI-R9 in a 5'-to-3' direction; e) cleaving, with a restriction endonuclease, the fifth polynucleotide, the sixth polynucleotide, the fifth vector polynucleotide and the sixth vector polynucleotide to obtain the cleaved fifth polynucleotide, the cleaved sixth polynucleotide, the cleaved fifth vector polynucleotide and the cleaved sixth vector polynucleotide; f) mixing the cleaved fifth polynucleotide, the cleaved sixth polynucleotide, the cleaved fifth vector polynucleotide and the cleaved sixth vector polynucleotide, such that the cleaved fifth polynucleotide, the cleaved sixth polynucleotide, the cleaved fifth vector polynucleotide and the cleaved sixth vector polynucleotide can be cyclized by directional linkage to form second to-be-screened vectors; g) expressing the second to-be-screened vectors, and selecting a second to-be-screened vector capable of expressing an expression product having the following property, as a second replacement vector: capability of binding to a target, to which the reference antigen-binding protein can bind, at a capacity that is 30% or more of a binding capacity of the reference antigen-binding protein to the target; and h) selecting a nucleic acid fragment II of the second replacement vector as the nucleic acid fragment II', wherein R9, R10, R11 and R12 are each independently a restriction endonuclease recognition site.

In some embodiments, the method includes cleaving the fifth polynucleotide with a restriction endonuclease specifically recognizing R9 and R10, to obtain the cleaved fifth polynucleotide.

In some embodiments, the method includes cleaving the sixth polynucleotide with a restriction endonuclease specifically recognizing R11 and R12, to obtain the cleaved sixth polynucleotide.

In some embodiments, the method includes cleaving the fifth vector polynucleotide with a restriction endonuclease specifically recognizing R10 and R11, to obtain the cleaved fifth vector polynucleotide.

In some embodiments, the method includes cleaving the sixth vector polynucleotide with a restriction endonuclease specifically recognizing R12 and R9, to obtain the cleaved sixth vector polynucleotide.

In some embodiments, a terminus of R9 resulting from specific cleavage by the restriction endonuclease specifically recognizing R9 and a terminus of any one of R10, R11 and R12 resulting from specific cleavage by the corresponding restriction endonuclease do not recognize or link to each other.

In some embodiments, a terminus of R10 resulting from specific cleavage by the restriction endonuclease specifically recognizing R10 and a terminus of any one of R11, R12 and R9 resulting from specific cleavage by the corresponding restriction endonuclease do not recognize or link to each other.

In some embodiments, a terminus of R11 resulting from specific cleavage by the restriction endonuclease specifically recognizing R11 and a terminus of any one of R9, R10 and R12 resulting from specific cleavage by the corresponding restriction endonuclease do not recognize or link to each other.

In some embodiments, a terminus of R12 resulting from specific cleavage by the restriction endonuclease specifically recognizing R12 and a terminus of any one of R9, R10 and R11 resulting from specific cleavage by the corresponding restriction endonuclease do not recognize or link to each other.

In some embodiments, the restriction endonuclease is selected from the group consisting of SfiI and BsmBI.

In some embodiments, the method includes introducing the second to-be-screened vector into a cell to express the second to-be-screened vector.

In some embodiments, the method includes introducing the second to-be-screened vector into a bacterium to prepare a phage library including one or more of the second to-be-screened vectors, thereby obtaining the functional antigen-binding protein from the phage library.

In some embodiments, the vector fragment V includes a linker, and the vector fragment IV is derived from a display vector.

In some embodiments, the reference nucleic acid fragment I encodes an antibody light chain or a fragment thereof; the vector fragment V includes the linker; the nucleic acid fragment II encodes an antibody heavy chain or a fragment thereof; and the vector fragment VI is derived from the display vector.

In some embodiments, R9 includes a nucleotide sequence as set forth in SEQ ID NO: 1.

In some embodiments, R10 includes a nucleotide sequence as set forth in SEQ ID NO: 2.

In some embodiments, R11 includes a nucleotide sequence as set forth in SEQ ID NO: 3.

In some embodiments, R12 includes a nucleotide sequence as set forth in SEQ ID NO: 4.

In some embodiments, the vector fragment VI includes a linker, and the vector fragment V is derived from a display vector.

In some embodiments, the reference nucleic acid fragment I encodes an antibody heavy chain or a fragment thereof; the vector fragment V is derived from the display vector; the nucleic acid fragment II encodes an antibody light chain or a fragment thereof; and the vector fragment VI includes the linker.

In some embodiments, R9 includes a nucleotide sequence as set forth in SEQ ID NO: 3.

In some embodiments, R10 includes a nucleotide sequence as set forth in SEQ ID NO: 4.

In some embodiments, R11 includes a nucleotide sequence as set forth in SEQ ID NO: 1.

In some embodiments, R12 includes a nucleotide sequence as set forth in SEQ ID NO: 2.

In some embodiments, the display vector is derived from a pComb3x vector.

In some embodiments, the linker includes a nucleic acid sequence encoding a signal peptide pelB or a fragment thereof.

In some embodiments, the linker has a length of about 50 bases to about 200 bases.

In some embodiments, the method includes introducing the second to-be-screened vector into a bacterium to obtain a DNA of the second to-be-screened vector from the bacterium, and introducing the DNA of the second to-be-screened vector into a cell, from which a functional antigen-specific binding polypeptide is obtained.

In some embodiments, the cell is a mammalian cell.

In some embodiments, the vector fragment V and/or the vector fragment VI are/is derived from a mammalian expression vector.

In some embodiments, the mammalian expression vector is derived from pDGB4.

In some embodiments, the reference nucleic acid fragment I encodes an antibody light chain or a fragment thereof; and the reference nucleic acid fragment II encodes an antibody heavy chain or a fragment thereof.

In some embodiments, R9 includes a nucleotide sequence as set forth in SEQ ID NO: 7.

In some embodiments, R10 includes a nucleotide sequence as set forth in SEQ ID NO: 8.

In some embodiments, R11 includes a nucleotide sequence as set forth in SEQ ID NO: 5.

In some embodiments, R12 includes a nucleotide sequence as set forth in SEQ ID NO: 6.

In some embodiments, the reference nucleic acid fragment I encodes an antibody heavy chain or a fragment thereof; and the nucleic acid fragment II encodes an antibody light chain or a fragment thereof.

In some embodiments, R9 includes a nucleotide sequence as set forth in SEQ ID NO: 5.

In some embodiments, R10 includes a nucleotide sequence as set forth in SEQ ID NO: 6.

In some embodiments, R11 includes a nucleotide sequence as set forth in SEQ ID NO: 7.

In some embodiments, R12 includes a nucleotide sequence as set forth in SEQ ID NO: 8.

In some embodiments, the antigen-binding protein includes an antibody or an antibody fragment.

In some embodiments, the antibody fragment includes scFv, Fab, Fab', (Fab)₂ and/or (Fab')₂.

In some embodiments, the directional linkage includes the use of a ligase.

In some embodiments, the ligase is a DNA ligase.

In another aspect, the present application provides a method for preparing an antigen-binding protein, including expressing the first replacement vector, the second replacement vector, and/or the double-replacement vector, under conditions allowing expression of the first replacement vector, the second replacement vector, and/or the double-replacement vector.

In another aspect, the present application provides a first replacement vector prepared according to the method as defined.

In another aspect, the present application provides a second replacement vector prepared according to the method as defined.

In another aspect, the present application provides a double replacement vector prepared according to the method as defined.

In another aspect, the present application provides a functional antigen-binding protein prepared according to the method as defined.

### BRIEF SUMMARY OF THE INVENTION

The present application provides a method for selecting a functional antigen-binding protein. The method includes constructing a vector (for example, a double-replacement to-be-screened vector, a first to-be-screened vector and/or a second to-be-screened vector) using a reference antigen-binding fragment (for example, a reference antigen-binding fragment I and/or a reference antigen-binding fragment II) of a reference antigen-binding protein with a known sequence and/or function to express the vector, and then obtaining the functional antigen-binding protein according to a binding activity of an expression product of the vector to a target recognizable by the reference antigen-binding protein. The present application further provides a method for constructing the vector, and a functional antigen-binding protein obtained by means of the vector. In the present application, a novel functional antigen-binding protein with desired properties is obtained by screening by means of a light and heavy chains, of fragments thereof, of the reference antigen-binding protein with a known sequence and/or function in the prior art, and in combination with the phage display technique or cell surface display technique for the antibody; and the functional antigen-binding protein obtained by screening has a high probability of binding to the same or similar epitope of the same target as the reference antigen-binding protein, showing high probability of biological activity, high screening efficiency, and short screening time.

Other aspects and advantages of the present application can be readily perceived by those skilled in the art from the detailed description below. The detailed description below only shows and describes the exemplary embodiments of the present application. As would be appreciated by those skilled in the art, the content of the present application enables those killed in the art to change the specific embodiments disclosed, without departing from the spirit and scope involved in the present application. Accordingly, the accompanying drawings and the description in the specification of the present application are merely for an exemplary but not restrictive purpose.

### BRIEF DESCRIPTION OF THE SEVERAL VIEWS OF THE DRAWINGS

The specific features of the invention involved in the present application are listed in the appended claims. The characteristics and advantages of the invention involved in the present application can be better understood by referring to the exemplary embodiments and the accompanying drawings described in detail below. The accompanying drawings are briefly illustrated as follows:
FIG. 1 shows an exemplary structure of a first to-be-screened vector according to the present application;
FIG. 2 shows an exemplary structure of a double-replacement to-be-screened vector according to the present application;
FIG. 3 shows an exemplary structure of a second to-be-screened vector according to the present application;
FIG. 4 shows the results of excitation activity analysis of antibodies obtained by the method of the present application;
FIG. 5 shows the results of endocytosis activity analysis of antibodies obtained by the method of the present application;
FIG. 6 shows an anti-tumor effect of individual administration of antibodies obtained by the method of the present application; and
FIG. 7 shows an anti-tumor effect of combined administration of antibodies obtained by the method of the present application.

### DETAILED DESCRIPTION OF THE INVENTION

The embodiments of the invention of the present application will be illustrated by specific examples below. Those familiar with this technology can easily understand other advantages and effects of the invention of the present application from the content disclosed in the specification.

### TERMS & DEFINITIONS

In the present application, the term "reference antigen-binding protein" generally refers to an antigen-binding protein having at least one of the desired biological functions, for example, capability of specifically binding to a target at high affinity, capability of blocking the binding of a protein to a specific ligand, and/or capability of stimulating immune cells to secrete cytokines, or the like. The reference antigen-binding protein may be an antigen-binding protein (for example, antibody) with a known amino acid sequence and/or function in the prior art. In some embodiments, the reference antigen-binding protein is sourced from a commercial antibody product and/or an antibody in clinical tests. The selection of reference antigen-binding protein (and "reference antigen-binding fragment I" and/or "reference antigen-binding fragment II") depends on the desired biological function of the antigen-binding protein. For example, if an antigen-binding protein specifically binding to PD-1 is desired, a PD-1 antibody Nivolumab, Pembrolizumab or Cemiplimab may be selected as a reference antigen-binding protein. For example, if an antigen-binding protein specifically binding to PD-L1 is desired, a PD-1 antibody Atezolizumab, Avelumab or Durvalumab may be selected as a reference antigen-binding protein.

In the present application, the term "reference nucleic acid fragment I" generally refers to a nucleic acid fragment capable of encoding a reference antigen-binding fragment I.

In the present application, the term "reference nucleic acid fragment II" generally refers to a nucleic acid fragment capable of encoding a reference antigen-binding fragment II.

In the present application, the term "reference antigen-binding fragment I" generally refers to a polypeptide fragment capable of forming a reference antigen-binding protein. The "reference antigen-binding fragment I" may be an antibody heavy chain, heavy-chain variable region, or heavy chain fragment, and may also be an antibody light chain, light-chain variable region, or light chain fragment.

In the present application, the term "reference antigen-binding fragment II" generally refers to a polypeptide fragment capable of forming a reference antigen-binding protein. The "reference antigen-binding fragment II" may be an antibody heavy chain, heavy-chain variable region, or heavy chain fragment, and may also be an antibody light chain, light-chain variable region, or light chain fragment.

The "reference antigen-binding fragment I" may form, together with the "reference antigen-binding fragment II", an antigen-binding protein (for example, a reference antigen-binding protein).

In the present application, the term "antigen-binding protein" generally refers to a protein containing an antigen-binding moiety, and optionally a scaffold or skeleton moiety that allows the antigen-binding moiety to adopt a conformation that promotes the binding of the antigen-binding protein to the antigen. The antigen-binding protein may typically include an antibody light-chain variable region (VL), an antibody heavy-chain variable region (VH) or both, and a functional fragment thereof. The heavy-chain and light-chain variable regions each include a binding domain that interacts with the antigen. Examples of the antigen-binding protein include, but are not limited to, antibodies, antigen-binding fragments, immunoconjugates, multispecific antibodies (for example, bispecific antibodies), antibody fragments, antibody derivatives, antibody analogs, or fusion proteins, etc., as long as they exhibit the desired antigen-binding activity.

In the present application, the term "antibody" generally refers to an immunoglobulin reactive to a specified protein or peptide or a fragment thereof. The antibody may be an antibody from any class (including but not limited to IgG, IgA, IgM, IgD and IgE), and an antibody from any subclass (for example, IgG1, IgG2, IgG3, and IgG4). The antibody may have a heavy-chain constant region selected from, for example, IgG1, IgG2, IgG3, or IgG4. The antibody may also have a light chain selected from, for example, kappa (κ) or lambda (λ). The antibody of the present application may be derived from any species.

In the present application, the term "antigen-binding protein" may refer to a moiety of an antibody molecule, and the moiety includes an amino acid residue which interacts with an antigen and endows the antibody with specificity and affinity to the antigen. In the present application, the term "antigen-binding protein" may include an antibody or an antibody fragment, in particular those antibody moieties including an antibody light chain or a fragment thereof (for example VL) and an antibody heavy chain or a fragment thereof (for example, VH). In the antigen-binding protein, the antibody light chain or the fragment thereof (for example, VL) and the antibody heavy chain or the fragment thereof (for example, VH) may be linked at an N-terminus or a C-terminus by means of a linker (for example, a peptide linker), to form a polypeptide chain. In the antigen-binding protein, the antibody light chain or the fragment thereof (for example, VL) and the antibody heavy chain or the fragment thereof (for example, VH) may also be linked by means of an interchain chemical bond (for example, a disulfide bond), to form a dimer. Examples of the antigen-binding protein may include, but not limited to, Fab, Fab', F(ab)₂, a Fv fragment, a F(ab')₂, scFv, di-scFv, and/or dAb. For example, the antigen-binding protein may be scFv or Fab.

In the present application, the term "Fab" generally refers to two identical antigen-binding fragments produced by digesting, by papain, an antibody having an intact structure (for example, with Fc and hinge regions removed). Fab may consist of a complete a light chain, a heavy-chain variable region (VH) and a heavy chain first constant domain (CH1). Each Fab may have a single antigen-binding site.

In the present application, the term "scFv" generally refers to a monovalent molecule produced by covalently linking and pairing one heavy-chain variable domain with one light-chain variable domain of an antibody by means of a flexible peptide linker.

In the present application, the term "functional antigen-binding protein" generally refers to an antigen-binding protein that is selected according to the method of the present application and has a desired function, for example, capability of specifically binding to a target at high affinity, capability of blocking the binding of a protein to a specific ligand, and/or capability of stimulating immune cells to secrete cytokines, or the like. The functional antigen-binding protein has the following property: capability of binding to a target, to which the reference antigen-binding protein of the present application may bind, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target. The functional antigen-binding protein may include a reference antigen-binding fragment I and an antigen-binding fragment II'. The functional antigen-binding protein may include a reference antigen-binding fragment II and an antigen-binding fragment I'. The functional antigen-binding protein may include an antigen-binding fragment I' and an antigen-binding fragment II' .

In the present application, the term "nucleic acid fragment I" generally refers to a nucleic acid molecule capable of expressing an antigen-binding fragment I. For the nucleic acid fragment I, a nucleic acid fragment I having the following property can be selected as a nucleic acid fragment I': expression of an antigen-binding fragment I capable of forming, together with a reference antigen-binding fragment I expressed by a reference nucleic acid fragment II, an antigen-binding protein, which has one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target. Not every nucleic acid fragment I is a nucleic acid fragment I'. Not every antigen-binding fragment I expressed by the nucleic acid fragment I is capable of forming, together with a reference antigen-binding fragment II expressed by the reference nucleic acid fragment II, an antigen-binding protein.

In the present application, the term "nucleic acid fragment I' " generally refers to a nucleic acid molecule capable of expressing an antigen-binding fragment I'. The antigen-binding fragment I' expressed by the nucleic acid fragment I' is capable of forming, together with the reference antigen-binding fragment I expressed by the reference nucleic acid fragment II, a functional antigen-binding protein, which has one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target.

In the present application, the term "nucleic acid fragment II" generally refers to a nucleic acid molecule capable of expressing an antigen-binding fragment II. For the nucleic acid fragment II, a nucleic acid fragment II having the following property can be selected as a nucleic acid fragment II': expression of an antigen-binding fragment II capable of forming, together with a reference antigen-binding fragment I expressed by a reference nucleic acid fragment I, an antigen-binding protein, which has one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target. Not every nucleic acid fragment II is a nucleic acid fragment II'. Not every antigen-binding fragment II expressed by the nucleic acid fragment II is capable of forming, together with a reference antigen-binding fragment I expressed by the reference nucleic acid fragment I, an antigen-binding protein.

In the present application, the term "nucleic acid fragment II' " generally refers to a nucleic acid molecule capable of expressing an antigen-binding fragment II'. The antigen-binding fragment II' expressed by the nucleic acid fragment II' is capable of forming, together with the reference antigen-binding fragment I expressed by the reference nucleic acid fragment I, a functional antigen-binding protein, which has one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target.

In the present application, the term "antigen-binding fragment I" generally refers to an expression product of the nucleic acid fragment I, and is encoded by the nucleic acid fragment I. For the antigen-binding fragment I, an antigen-binding fragment I having the following property can be selected as an antigen-binding fragment I': capability of forming, together with a reference antigen-binding fragment II, an antigen-binding protein, which has one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target. Not every antigen-binding fragment I is an antigen-binding fragment I'. The "antigen-binding fragment I" may be an antibody heavy chain, heavy-chain variable region, or heavy chain fragment, and may also be an antibody light chain, light-chain variable region, or light chain fragment.

In the present application, the term "antigen-binding fragment I' " generally refers to an expression product of the nucleic acid fragment I', and is encoded by the nucleic acid fragment I'. The antigen-binding fragment I' is an antigen-binding fragment I capable of forming, together with a reference antigen-binding fragment II, a functional antigen-binding protein, which has one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target.

In the present application, the term "antigen-binding fragment II" generally refers to an expression product of the nucleic acid fragment II, and is encoded by the nucleic acid fragment II. For the antigen-binding fragment II, an antigen-binding fragment II having the following property can be selected as an antigen-binding fragment II': capability of forming, together with a reference antigen-binding fragment I, an antigen-binding protein, which has one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target. Not every antigen-binding fragment II is an antigen-binding fragment II". The "antigen-binding fragment II" may be an antibody heavy chain, heavy-chain variable region, or heavy chain fragment, and may also be an antibody light chain, light-chain variable region, or light chain fragment.

In the present application, the term "antigen-binding fragment II" generally refers to an expression product of the nucleic acid fragment II', and is encoded by the nucleic acid fragment II'. The antigen-binding fragment II' is an antigen-binding fragment II capable of forming, together with a reference antigen-binding fragment I, a functional antigen-binding protein, which has one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target.

In the present application, the term "first polynucleotide" generally refers to a polynucleotide including a nucleic acid fragment I. In the present application, the first polynucleotide may also have an endonuclease (for example, restriction endonuclease) recognition site present at a 5'-terminus and/or 3'-terminus. For example, the first polynucleotide may include a structure R1-nucleic acid fragment I-R2 in a 5'-to-3' direction, and R1 and R2 may be restriction endonuclease recognition sites. For example, after the digestion by an endonuclease (for example, a restriction endonuclease recognizing R1 and R2, for example, SfiI and BsmBI) recognizing the endonuclease recognition site in the first polynucleotide, the digested first polynucleotide may include the nucleic acid fragment I.

In the present application, the term "first vector polynucleotide" generally refers to a polynucleotide including a vector fragment I. In the present application, the first vector polynucleotide may also have an endonuclease (for example, restriction endonuclease) recognition site present at a 5'-terminus and/or 3'-terminus. For example, the first vector polynucleotide may include a structure R2-vector fragment I-R3 in a 5'-to-3' direction, and R2 and R3 may be restriction endonuclease recognition sites. For example, after the digestion by an endonuclease (for example, a restriction endonuclease recognizing R2 and R3, for example, SfiI and BsmBI) recognizing the endonuclease recognition site in the first polynucleotide, the digested first vector polynucleotide may include the vector fragment I.

In the present application, the term "second polynucleotide" generally refers to a polynucleotide including a reference nucleic acid fragment II. In the present application, the second polynucleotide may also have an endonuclease (for example, restriction endonuclease) recognition site present at a 5'-terminus and/or 3'-terminus. For example, the second polynucleotide may include a structure R3-reference nucleic acid fragment II-R4 in a 5'-to-3' direction, and R3 and R4 may be restriction endonuclease recognition sites. For example, after the digestion by an endonuclease (for example, a restriction endonuclease recognizing R3 and R4, for example, SfiI and BsmBI) recognizing the endonuclease recognition site in the second polynucleotide, the digested second polynucleotide may include the reference nucleic acid fragment II.

In the present application, the term "second vector polynucleotide" generally refers to a polynucleotide including a vector fragment II. In the present application, the second polynucleotide may also have an endonuclease (for example, restriction endonuclease) recognition site present at a 5'-terminus and/or 3'-terminus. For example, the second vector polynucleotide may include a structure R4-vector fragment II-R1 in a 5'-to-3' direction, and R4 and R1 may be restriction endonuclease recognition sites. For example, after the digestion by an endonuclease (for example, a restriction endonuclease recognizing R4 and R1, for example, SfiI and BsmBI) recognizing the endonuclease recognition site in the second polynucleotide, the digested second polynucleotide may include the vector fragment II.

In the present application, the term "third polynucleotide" generally refers to a polynucleotide including a nucleic acid fragment I'. In the present application, the third polynucleotide may also have an endonuclease (for example, restriction endonuclease) recognition site present at a 5'-terminus and/or 3'-terminus. For example, the third polynucleotide may include a structure R5-nucleic acid fragment I'-R6 in a 5'-to-3' direction, and R5 and R6 may be restriction endonuclease recognition sites. For example, after the digestion by an endonuclease (for example, a restriction endonuclease recognizing R5 and R6, for example, SfiI and BsmBI) recognizing the endonuclease recognition site in the third polynucleotide, the digested third polynucleotide may include the nucleic acid fragment I' .

In the present application, the term "third vector polynucleotide" generally refers to a polynucleotide including a vector fragment III. In the present application, the third vector polynucleotide may also have an endonuclease (for example, restriction endonuclease) recognition site present at a 5'-terminus and/or 3'-terminus. For example, the third vector polynucleotide may include a structure R6-vector fragment III-R7 in a 5'-to-3' direction, and R6 and R7 may be restriction endonuclease recognition sites. For example, after the digestion by an endonuclease (for example, a restriction endonuclease recognizing R6 and R7, for example, SfiI and BsmBI) recognizing the endonuclease recognition site in the third vector polynucleotide, the digested third vector polynucleotide may include the vector fragment III.

In the present application, the term "fourth polynucleotide" generally refers to a polynucleotide including a nucleic acid fragment II'. In the present application, the fourth polynucleotide may also have an endonuclease (for example, restriction endonuclease) recognition site present at a 5'-terminus and/or 3'-terminus. For example, the fourth polynucleotide may include a structure R7-nucleic acid fragment II'-R8 in a 5'-to-3' direction, and R7 and R8 may be restriction endonuclease recognition sites. For example, after the digestion by an endonuclease (for example, a restriction endonuclease recognizing R7 and R8, for example, SfiI and BsmBI) recognizing the endonuclease recognition site in the fourth polynucleotide, the digested second polynucleotide may include the reference nucleic acid fragment II'.

In the present application, the term "fourth vector polynucleotide" generally refers to a polynucleotide including a vector fragment IV In the present application, the fourth vector polynucleotide may also have an endonuclease (for example, restriction endonuclease) recognition site present at a 5'-terminus and/or 3'-terminus. For example, the fourth vector polynucleotide may include a structure R8-vector fragment IV-R5 in a 5'-to-3' direction, and R8 and R5 may be restriction endonuclease recognition sites. For example, after the digestion by an endonuclease (for example, a restriction endonuclease recognizing R8 and R5, for example, SfiI and BsmBI) recognizing the endonuclease recognition site in the fourth vector polynucleotide, the digested fourth vector polynucleotide may include the vector fragment IV

In the present application, the term "fifth polynucleotide" generally refers to a polynucleotide including a reference nucleic acid fragment I. In the present application, the fifth polynucleotide may also have an endonuclease (for example, restriction endonuclease) recognition site present at a 5'-terminus and/or 3'-terminus. For example, the fifth polynucleotide may include a structure R9-reference nucleic acid fragment I-R10 in a 5'-to-3' direction, and R9 and R10 may be restriction endonuclease recognition sites. For example, after the digestion by an endonuclease (for example, a restriction endonuclease recognizing R9 and R10, for example, SfiI and BsmBI) recognizing the endonuclease recognition site in the fifth polynucleotide, the digested fifth polynucleotide may include the reference nucleic acid fragment I.

In the present application, the term "fifth vector polynucleotide" generally refers to a polynucleotide including a vector fragment V In the present application, the fifth vector polynucleotide may also have an endonuclease (for example, restriction endonuclease) recognition site present at a 5'-terminus and/or 3'-terminus. For example, the fifth vector polynucleotide may include a structure R10-vector fragment V-R11 in a 5'-to-3' direction, and R10 and R11 may be restriction endonuclease recognition sites. For example, after the digestion by an endonuclease (for example, a restriction endonuclease recognizing R10 and R11, for example, SfiI and BsmBI) recognizing the endonuclease recognition site in the fifth vector polynucleotide, the digested fifth vector polynucleotide may include the vector fragment V

In the present application, the term "sixth polynucleotide" generally refers to a polynucleotide including a nucleic acid fragment II. In the present application, the sixth polynucleotide may also have an endonuclease (for example, restriction endonuclease) recognition site present at a 5'-terminus and/or 3'-terminus. For example, the sixth polynucleotide may include a structure R11-nucleic acid fragment II-R12 in a 5'-to-3' direction, and R11 and R12 may be restriction endonuclease recognition sites. For example, after the digestion by an endonuclease (for example, a restriction endonuclease recognizing R11 and R12, for example, SfiI and BsmBI) recognizing the endonuclease recognition site in the sixth polynucleotide, the digested sixth polynucleotide may include the reference nucleic acid fragment II.

In the present application, the term "sixth vector polynucleotide" generally refers to a polynucleotide including a vector fragment VI. In the present application, the sixth vector polynucleotide may also have an endonuclease (for example, restriction endonuclease) recognition site present at a 5'-terminus and/or 3'-terminus. For example, the sixth vector polynucleotide may include a structure R12-vector fragment VI-R9 in a 5'-to-3' direction, and R12 and R9 may be restriction endonuclease recognition sites. For example, after the digestion by an endonuclease (for example, a restriction endonuclease recognizing R12 and R9, for example, SfiI and BsmBI) recognizing the endonuclease recognition site in the sixth vector polynucleotide, the digested sixth vector polynucleotide may include the vector fragment VI.

In the present application, the term "cleaved first polynucleotide" generally refers to a nucleic acid molecule obtained by treating the first polynucleotide with a restriction endonuclease. The "cleaved first polynucleotide" includes a nucleic acid fragment I, and termini, at two ends, which are obtained by treatment with a restriction endonuclease and have specific sequences.

In the present application, the term "cleaved first vector polynucleotide" generally refers to a nucleic acid molecule obtained by treating the first vector polynucleotide with a restriction endonuclease. The "cleaved first vector polynucleotide" includes a vector fragment I, and termini, at two ends, which are obtained by treatment with a restriction endonuclease and have specific sequences.

In the present application, the term "cleaved second polynucleotide" generally refers to a nucleic acid molecule obtained by treating the second polynucleotide with a restriction endonuclease. The "cleaved second polynucleotide" includes a reference nucleic acid fragment II, and termini, at two ends, which are obtained by treatment with a restriction endonuclease and have specific sequences.

In the present application, the term "cleaved second vector polynucleotide" generally refers to a nucleic acid molecule obtained by treating the second vector polynucleotide with a restriction endonuclease. The "cleaved second vector polynucleotide" includes a vector fragment II, and termini, at two ends, which are obtained by treatment with a restriction endonuclease and have specific sequences.

In the present application, the term "cleaved third polynucleotide" generally refers to a nucleic acid molecule obtained by treating the third polynucleotide with a restriction endonuclease. The "cleaved third polynucleotide" includes a nucleic acid fragment I', and termini, at two ends, which are obtained by treatment with a restriction endonuclease and have specific sequences.

In the present application, the term "cleaved third vector polynucleotide" generally refers to a nucleic acid molecule obtained by treating the third vector polynucleotide with a restriction endonuclease. The "cleaved third vector polynucleotide" includes a vector fragment III, and termini, at two ends, which are obtained by treatment with a restriction endonuclease and have specific sequences.

In the present application, the term "cleaved fourth polynucleotide" generally refers to a nucleic acid molecule obtained by treating the fourth polynucleotide with a restriction endonuclease. The "cleaved fourth polynucleotide" includes a reference nucleic acid fragment II', and termini, at two ends, which are obtained by treatment with a restriction endonuclease and have specific sequences.

In the present application, the term "cleaved fourth vector polynucleotide" generally refers to a nucleic acid molecule obtained by treating the fourth vector polynucleotide with a restriction endonuclease. The "cleaved fourth vector polynucleotide" includes a vector fragment IV, and termini, at two ends, which are obtained by treatment with a restriction endonuclease and have specific sequences.

In the present application, the term "cleaved fifth polynucleotide" generally refers to a nucleic acid molecule obtained by treating the fifth polynucleotide with a restriction endonuclease. The "cleaved fifth polynucleotide" includes a reference nucleic acid fragment I, and termini, at two ends, which are obtained by treatment with a restriction endonuclease and have specific sequences.

In the present application, the term "cleaved fifth vector polynucleotide" generally refers to a nucleic acid molecule obtained by treating the fifth vector polynucleotide with a restriction endonuclease. The "cleaved fifth vector polynucleotide" includes a vector fragment V, and termini, at two ends, which are obtained by treatment with a restriction endonuclease and have specific sequences.

In the present application, the term "cleaved sixth polynucleotide" generally refers to a nucleic acid molecule obtained by treating the sixth polynucleotide with a restriction endonuclease. The "cleaved sixth polynucleotide" includes a nucleic acid fragment II, and termini, at two ends, which are obtained by treatment with a restriction endonuclease and have specific sequences.

In the present application, the term "cleaved sixth vector polynucleotide" generally refers to a nucleic acid molecule obtained by treating the sixth vector polynucleotide with a restriction endonuclease. The "cleaved sixth vector polynucleotide" includes a vector fragment VI, and termini, at two ends, which are obtained by treatment with a restriction endonuclease and have specific sequences.

In the present application, the term "first to-be-screened vector" generally refers to a cyclic nucleic acid molecule including a nucleic acid fragment I, a reference nucleic acid fragment II, a vector fragment I and a vector fragment II. In some embodiments, the "first to-be-screened vector" is formed by directional linkage of the cleaved first polynucleotide, the cleaved second polynucleotide, the cleaved first vector polynucleotide and the cleaved second vector polynucleotide. In some cases, the "first to-be-screened vector" is capable of expressing and forming an antigen-binding protein. In other cases, the "first to-be-screened vector" is incapable of expressing and forming an antigen-binding protein. When the antigen-binding protein expressed and formed by the "first to-be-screened vector" has one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target, the "first to-be-screened vector" may be referred to as a "first replacement vector", or a nucleic acid fragment I in the "first to-be-screened vector" may be referred to as a nucleic acid fragment I'. A "first to-be-screened vector library" includes one or more first to-be-screened vectors.

In the present application, the term "first replacement vector" generally refers to a cyclic nucleic acid molecule including a nucleic acid fragment I', a reference nucleic acid fragment II, a vector fragment I and a vector fragment II. The "first replacement vector" may express and form an antigen-binding protein, which has one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target. The "first to-be-screened vector library" includes one or more "first to-be-screened vectors", but excludes those first to-be-screened vectors expressing and forming antigen-binding proteins that do not have one or more of the following properties: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target.

In the present application, the term "second to-be-screened vector" generally refers to a cyclic nucleic acid molecule including a nucleic acid fragment II, a reference nucleic acid fragment I, a vector fragment V and a vector fragment VI. In some embodiments, the "second to-be-screened vector" is formed by directional linkage of the cleaved fifth polynucleotide, the cleaved sixth polynucleotide, the cleaved fifth vector polynucleotide and the cleaved sixth vector polynucleotide. The "second to-be-screened vector" is capable of expressing and forming an antigen-binding protein. When the antigen-binding protein expressed and formed by the "second to-be-screened vector" has the following property: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target, the "second to-be-screened vector" may be referred to as a "second replacement vector", or a nucleic acid fragment II in the "second to-be-screened vector" may be referred to as a nucleic acid fragment II'. A "second to-be-screened vector library" includes one or more second to-be-screened vectors.

In the present application, the term "second replacement vector" generally refers to a cyclic nucleic acid molecule including a nucleic acid fragment II', a reference nucleic acid fragment I, a vector fragment V and a vector fragment VI. The "second replacement vector" may express and form an antigen-binding protein, which has the following property: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target. In the present application, the "second replacement vector library" includes one or more "second replacement vectors", but excludes those second to-be-screened vectors expressing and forming antigen-binding proteins that do not have the following property: capability of binding to a target, to which the reference antigen-binding protein specifically binds, at a capacity that is 30% or more (for example, 35%, 40%, 45%, 50%, 55%, 60%, 65%, 70%, 75%, 80%, 85%, 90%, 95% or 99% or more) of a binding capacity of the reference antigen-binding protein to the target.

In the present application, the term "double-replacement to-be-screened vector" generally refers to a cyclic nucleic acid molecule including a nucleic acid fragment I', a nucleic acid fragment II', a vector fragment I and a vector fragment II. The "double-replacement to-be-screened vector" is generally formed by directional linkage of the cleaved first polynucleotide, the cleaved second polynucleotide, the cleaved first vector polynucleotide and the cleaved second vector polynucleotide. In some cases, the "double-replacement to-be-screened vector" is capable of expressing and forming an antigen-binding protein. In other cases, the "double-replacement to-be-screened vector" is incapable of expressing and forming an antigen-binding protein. If an expression product of the double-replacement to-be-screened vector has the following property: capability of binding to a target, to which the reference antigen-binding protein specifically binds, at a capacity that is 30% or more of (for example, 35% or more, 40% or more, 45% or more, 50% or more, 55% or more, 60% or more, 65% or more, 70% or more, 75% or more, 80% or more, 85% or more, 90% or more, 95% or more, 99% or more of, or 1 time or more, 1.5 times or more, 2 times or more, 2.5 times or more, 3 times or more, or above) a binding capacity of the reference antigen-binding protein to the target, the double-replacement to-be-screened vector may be referred to as a double-replacement vector. A "double-replacement to-be-screened vector library" includes one or more "double-replacement to-be-screened vectors".

In the present application, the term "cleavage" generally refers to allowing contact between a restriction endonuclease and a polynucleotide under a condition enabling cleavage. The cleavage generally refers to cleaving a bond between a carbohydrate molecule of a nucleotide and a phosphate of an adjacent nucleotide molecule. For example, the cleavage generally refers to cleaving a phosphate diester bond between two nucleotides. After the cleavage by the restriction endonuclease, the polynucleotide may produce termini having specific sequences.

In the present application, the term "linkage" generally refers to linking two or more polynucleotide molecules together. For example, said linkage may be achieved by a ligase (for example, a DNA ligase). For example, the 3'-terminus of one polynucleotide is linked to the 5'-terminus of another polynucleotide to form a complete polynucleotide molecule. "Directional linkage" generally refers to linking two or more polynucleotide molecules in a certain order.

In the present application, the term "introducing" generally refers to a process of transferring or importing an exogenous polynucleotide into a cell. The cell may be a host cell. The cell allowing introduction includes a primary cell of an object and a progeny thereof. The cell may be a prokaryotic cell, for example, a bacterial cell. The cell may be a eukaryotic cell, for example, a mammalian cell or a yeast cell.

In the present application, the term "linker" generally refers to a reagent capable of linking more than two polynucleotide molecules or fragments thereof. The linker may be a polynucleotide or a fragment thereof. In the present application, the linker may have different lengths. For example, the linker may have a length of 40 bp or more, 50 bp or more, 60 bp or more, 70 bp or more, 80 bp or more, 90 bp or more, 100 bp or more, 150 bp or more, 200 bp or more, or above. In some cases, the linker includes a signal peptide, and the term "signal peptide pelB" generally refers to a signal peptide of a pectinase lyase, for example, when an antigen-binding protein is Fab. The signal peptide pelB is generally used in a prokaryotic expression system. For example, the signal peptide pelB may have an accession number of ABS75961.1 in GenBank. In some cases, the linker does not include a signal peptide, for example, when an antigen-binding protein is scFv. In some cases, the first polynucleotide may include a nucleic acid molecule encoding VH, and the second polynucleotide may include a nucleic acid molecule encoding VL. In some cases, the first polynucleotide may include a nucleic acid molecule encoding VL, and the second polynucleotide may include a nucleic acid molecule encoding VH.

In the present application, the term "light chain or a fragment thereof' generally refers to an amino acid fragment capable of binding to a heavy chain of the same or similar antibody. In the present application, the light chain or the fragment thereof may include a light-chain variable region (VL) and a light-chain constant region (CL). The light-chain constant region may include κ and λ types. The light chain may further include a light chain having a λ variable region (V-λ) linked to a κ constant region (C-κ) or a κ variable region (V-κ) linked to a λ constant region (C-λ).

In the present application, the term "heavy chain or a fragment thereof' generally refers to an amino acid fragment capable of binding to a light chain of the same or similar antibody. In the present application, the heavy chain or the fragment thereof may be capable of binding to a light chain of the same or similar antibody. In the present application, the heavy-chain or heavy-chain fragment may include a heavy-chain variable region (VH) and a heavy-chain constant region (CH). The heavy-chain constant region may include a CH1 domain, a hinge region, a CH2 domain and a CH3 domain. In the cases of IgE, IgM and IgY, the heavy-chain constant region may include a CH4 domain, and does not include a hinge region. In the present application, the "heavy-chain constant region" may be a CH1 domain, a hinge region, a CH2 domain, a CH3 domain, a CH4 domain or any of their combinations.

In the present application, the term "restriction endonuclease" generally refers to an enzyme cleaving a double-strand DNA. The restriction endonuclease may produce a cohesive terminus having an overhang single-strand DNA, and thus may be cohered to a DNA ligase. In the present application, the restriction endonuclease may have effects such as recognition and restrictive cleavage. For example, a certain distance exists between a cleavage site of the restriction endonuclease and a recognition site of the restriction endonuclease. For example, the restriction endonuclease may be selected from the group consisting of SfiI, BsmBI and Esp3I.

In the present application, the term "polynucleotide" generally refers to nucleotides, i.e., at least two nucleotides that are linked together. The polynucleotide may be a polymer of any length, including, for example, 200, 300, 500, 1000, 2000, 3000, 5000, 7000, 10,000, and 100,000. The polynucleotide may include a phosphate diester bond.

In the present application, the term "comprise/include" generally refers to the inclusion of an explicitly specified feature, but not excluding other elements.

In the present application, the term "about" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, and 10% above or below a specified value.

### DETAILED DESCRIPTION OF THE INVENTION

The present application provides a method for selecting a functional antigen-binding protein by combining a chain replacement technique and an antibody display technique (for example, a phage display technique and a mammalian cell display technique).

### First Replacement

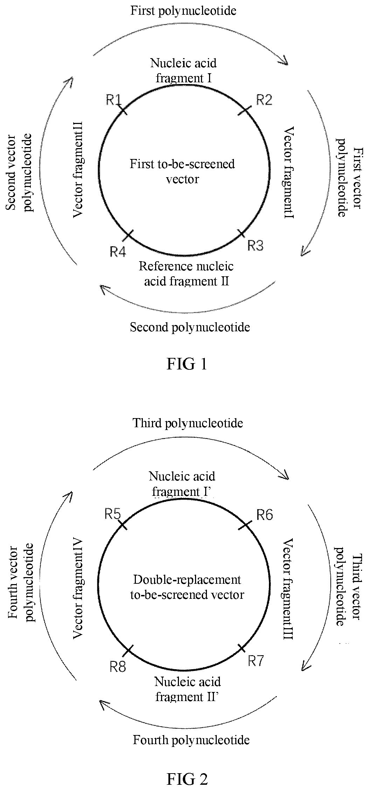

In the present application, the method includes constructing a to-be-screened library including a first to-be-screened vector by using an antibody heavy chain or a fragment thereof (or an antibody light chain or a fragment thereof) of a reference antigen-binding protein, and a plurality of antibody light chains and fragments thereof (or antibody heavy chains or fragments thereof). The first to-be-screened vector may be a phage display vector or a cell display vector, or any other display vector that can be selected according to the actual condition. Specifically, the method includes: a) providing a first polynucleotide including R1-nucleic acid fragment I-R2 in a 5'-to-3' direction, the nucleic acid fragment I being capable of encoding an antigen-binding fragment I; b) providing a second polynucleotide including R3-reference nucleic acid fragment II-R4 in a 5'-to-3' direction, the reference nucleic acid fragment II being capable of encoding a reference antigen-binding fragment II, which, together with a reference antigen-binding fragment I encoded by a reference nucleic acid fragment I, is capable of forming a reference antigen-binding protein; c) providing a first vector polynucleotide including R2-vector fragment I-R3 in a 5'-to-3' direction; d) providing a second vector polynucleotide including R4-vector fragment II-R1 in a 5'-to-3' direction; e) cleaving, with a restriction endonuclease, the first polynucleotide, the second polynucleotide, the first vector polynucleotide and the second vector polynucleotide to obtain the cleaved first polynucleotide, the cleaved second polynucleotide, the cleaved first vector polynucleotide and the cleaved second vector polynucleotide; and f) mixing the cleaved first polynucleotide, the cleaved second polynucleotide, the cleaved first vector polynucleotide and the cleaved second vector polynucleotide, such that the cleaved first polynucleotide, the cleaved second polynucleotide, the cleaved first vector polynucleotide and the cleaved second vector polynucleotide can be cyclized by directional linkage to form first to-be-screened vectors. The structure of the first to-be-screened vector may be found in FIG. 1. The method further includes: expressing the first to-be-screened vectors, and by comparison with a reference antigen-binding protein (or other positive control antibodies), selecting an antigen-binding protein having the following property, as the functional antigen-binding protein: capability of binding to a target, to which the reference antigen-binding protein can bind, at a capacity that is 30% or more of a binding capacity of the reference antigen-binding protein to the target.

In the present application, the nucleic acid fragment I may encode an antibody light chain or a fragment thereof; the reference nucleic acid fragment II may encode a heavy chain, or a fragment thereof, of a reference antigen-binding protein; the vector fragment I may include a linkage peptide; and the vector fragment II may be derived from a phage display vector (for example, a pComb3x vector). In some cases, the nucleic acid fragment I encodes an antibody light chain, and the linkage peptide includes a nucleic acid sequence encoding a signal peptide pelB or a fragment thereof. In some cases, the nucleic acid fragment I encodes an antibody light-chain variable region. R1 may include a nucleotide sequence as set forth in SEQ ID NO: 1, R2 may include a nucleotide sequence as set forth in SEQ ID NO: 2, R3 may include a nucleotide sequence as set forth in SEQ ID NO: 3, and R4 may include a nucleotide sequence as set forth in SEQ ID NO: 4.

In the present application, the nucleic acid fragment I may encode an antibody light chain or a fragment thereof; the reference nucleic acid fragment II may encode a heavy chain, or a fragment thereof, of a reference antigen-binding protein; and the vector fragment I and/or the vector fragment II may be derived from a mammalian cell expression vector (for example, a pDGB4 vector). R1 may include a nucleotide sequence as set forth in SEQ ID NO: 7, R2 may include a nucleotide sequence as set forth in SEQ ID NO: 8, R3 may include a nucleotide sequence as set forth in SEQ ID NO: 5, and R4 may include a nucleotide sequence as set forth in SEQ ID NO: 6.

In the present application, the nucleic acid fragment I may encode an antibody heavy chain or a fragment thereof; the reference nucleic acid fragment II may encode a light chain, or a fragment thereof, of a reference antigen-binding protein; the vector fragment II may include a linkage peptide; and the vector fragment I may be derived from a phage display vector (for example, a pComb3x vector). In some cases, the reference nucleic acid fragment II encodes an antibody light chain, and the linkage peptide includes a nucleic acid sequence encoding a signal peptide pelB or a fragment thereof. In some cases, the reference nucleic acid fragment II encodes an antibody light-chain variable region. R1 may include a nucleotide sequence as set forth in SEQ ID NO: 3, R2 may include a nucleotide sequence as set forth in SEQ ID NO: 4, R3 may include a nucleotide sequence as set forth in SEQ ID NO: 1, and R4 may include a nucleotide sequence as set forth in SEQ ID NO: 2.

In the present application, the nucleic acid fragment I may encode an antibody heavy chain or a fragment thereof; the reference nucleic acid fragment II may encode a light chain, or a fragment thereof, of a reference antigen-binding protein; and the vector fragment I and/or the vector fragment II may be derived from a mammalian cell expression vector (for example, a pDGB4 vector). R1 may include a nucleotide sequence as set forth in SEQ ID NO: 5, R2 may include a nucleotide sequence as set forth in SEQ ID NO: 6, R3 may include a nucleotide sequence as set forth in SEQ ID NO: 7, and R4 may include a nucleotide sequence as set forth in SEQ ID NO: 8.

The first to-be-screened vector capable of expressing a functional antigen-binding protein is referred to as a first replacement vector. Accordingly, the nucleic acid fragment I of the first to-be-screened vector capable of expressing the functional antigen-binding protein is referred to as a nucleic acid fragment I' of the first replacement vector.

### Second Replacement

The method of the present application may further include: after the nucleic acid fragment I' is obtained, constructing a double-replacement to-be-screened vector using the nucleic acid fragment I' with a nucleic acid fragment II'. The nucleic acid fragment II' may be derived by any method, for example by replacement with a reference antigen-binding fragment of the reference antigen-binding protein, in the art, as long as the following condition is satisfied: capability of encoding an antigen-binding protein II', which, together with the reference antigen-binding fragment I, is capable of forming an antigen-binding protein having the following property: capability of binding to a target, to which the reference antigen-binding protein can bind, at a capacity that is 30% or more of a binding capacity of the reference antigen-binding protein to the target.

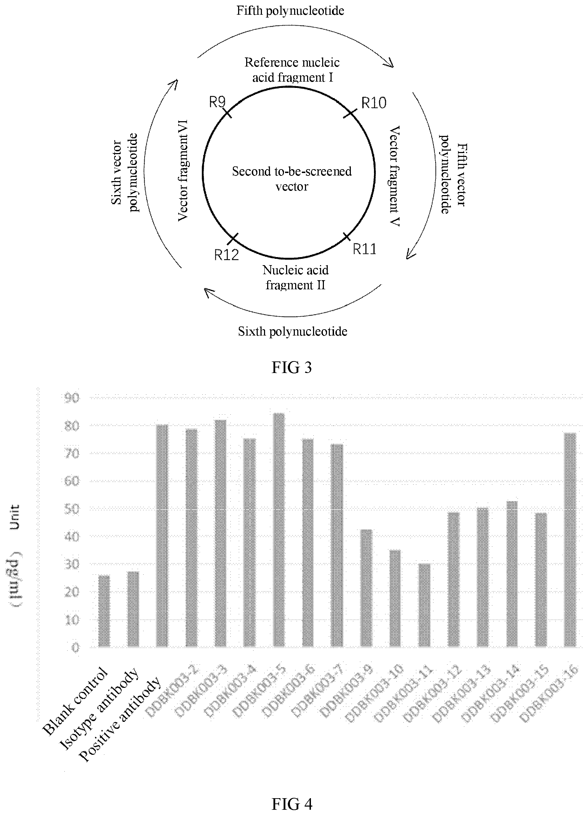

In some cases, the nucleic acid fragment II' is obtained by constructing a second to-be-screened vector. The method of the present application further includes: a) providing a fifth polynucleotide including R9-reference nucleic acid fragment I-R10 in a 5'-to-3' direction; b) providing a sixth polynucleotide including R11-nucleic acid fragment II-R12 in a 5'-to-3' direction; c) providing a fifth vector polynucleotide including R10-vector fragment V-R11 in a 5'-to-3' direction; d) providing a sixth vector polynucleotide including R12-vector fragment VI-R9 in a 5'-to-3' direction; e) cleaving, with a restriction endonuclease, the fifth polynucleotide, the sixth polynucleotide, the fifth vector polynucleotide and the sixth vector polynucleotide to obtain the cleaved fifth polynucleotide, the cleaved sixth polynucleotide, the cleaved fifth vector polynucleotide and the cleaved sixth vector polynucleotide; and f) mixing the cleaved fifth polynucleotide, the cleaved sixth polynucleotide, the cleaved fifth vector polynucleotide and the cleaved sixth vector polynucleotide, such that the cleaved fifth polynucleotide, the cleaved sixth polynucleotide, the cleaved fifth vector polynucleotide and the cleaved sixth vector polynucleotide can be cyclized by directional linkage to form second to-be-screened vectors. The structure of the second to-be-screened vector may be found in FIG. 3. The method further includes: expressing the first to-be-screened vectors, and by comparison with a reference antigen-binding protein (or other positive control antibodies), selecting an antigen-binding protein having the following property, as the functional antigen-binding protein: capability of binding to a target, to which the reference antigen-binding protein can bind, at a capacity that is 30% or more of a binding capacity of the reference antigen-binding protein to the target.

Whether the nucleic acid fragment II encodes an antibody light chain or a fragment thereof or encodes an antibody heavy chain or a fragment thereof depends on the type of the nucleic acid fragment I. As long as one of the polypeptides encoded by the nucleic acid fragment II and the polypeptide encoded by the nucleic acid fragment I is the antibody light chain or a fragment thereof, the other is the antibody heavy chain or a fragment thereof.

In the present application, the reference nucleic acid fragment I may encode an antibody light chain, or a fragment thereof, of a reference antigen-binding protein; the nucleic acid fragment II may encode an antibody heavy chain or a fragment thereof; the vector fragment V may include a linkage peptide; and the vector fragment VI may be derived from a phage display vector (for example, a pComb3x vector). In some cases, the reference nucleic acid fragment I encodes an antibody light chain, and the linkage peptide includes a nucleic acid sequence encoding a signal peptide pelB or a fragment thereof. In some cases, the reference nucleic acid fragment I encodes an antibody light-chain variable region. R9 may include a nucleotide sequence as set forth in SEQ ID NO: 1, R10 may include a nucleotide sequence as set forth in SEQ ID NO: 2, R11 may include a nucleotide sequence as set forth in SEQ ID NO: 3, and R12 may include a nucleotide sequence as set forth in SEQ ID NO: 4.

In the present application, the reference nucleic acid fragment I may encode an antibody light chain, or a fragment thereof, of a reference antigen-binding protein; the nucleic acid fragment II may encode a heavy chain or a fragment thereof; and the vector fragment V and/or the vector fragment VI may be derived from a mammalian cell expression vector (for example, a pDGB4 vector). R9 may include a nucleotide sequence as set forth in SEQ ID NO: 7, R10 may include a nucleotide sequence as set forth in SEQ ID NO: 8, R11 may include a nucleotide sequence as set forth in SEQ ID NO: 5, and R12 may include a nucleotide sequence as set forth in SEQ ID NO: 6.

In the present application, the reference nucleic acid fragment I may encode an antibody heavy chain, or a fragment thereof, of a reference antigen-binding protein; the nucleic acid fragment II may encode a light chain, or a fragment thereof, of an antigen-binding protein; the vector fragment VI may include a linkage peptide; and the vector fragment V may be derived from a phage display vector (for example, a pComb3x vector). In some cases, the nucleic acid fragment II encodes an antibody light chain, and the linkage peptide includes a nucleic acid sequence encoding a signal peptide pelB or a fragment thereof. In some cases, the nucleic acid fragment II encodes an antibody light-chain variable region. R9 may include a nucleotide sequence as set forth in SEQ ID NO: 3, R10 may include a nucleotide sequence as set forth in SEQ ID NO: 4, R11 may include a nucleotide sequence as set forth in SEQ ID NO: 1, and R12 may include a nucleotide sequence as set forth in SEQ ID NO: 2.

In the present application, the reference nucleic acid fragment I may encode an antibody heavy chain or a fragment thereof; the nucleic acid fragment II may encode a light chain, or a fragment thereof, of a reference antigen-binding protein; and the vector fragment V and/or the vector fragment VI may be derived from a mammalian cell expression vector (for example, a pDGB4 vector). R9 may include a nucleotide sequence as set forth in SEQ ID NO: 5, R10 may include a nucleotide sequence as set forth in SEQ ID NO: 6, R11 may include a nucleotide sequence as set forth in SEQ ID NO: 7, and R12 may include a nucleotide sequence as set forth in SEQ ID NO: 8.

### Double Replacement

The method of the present application further includes: obtaining a nucleic acid fragment I' and a nucleic acid fragment II', with one of a polypeptide encoded by the nucleic acid fragment I' and a polypeptide encoded by the nucleic acid fragment II' being an antibody light chain or a fragment thereof, and the other being an antibody heavy chain or a fragment thereof; b) providing a third polynucleotide including R5-nucleic acid fragment I'-R6 in a 5'-to-3' direction; c) providing a fourth polynucleotide including R7-nucleic acid fragment II'-R8 in a 5'-to-3' direction, wherein the nucleic acid fragment II' is capable of encoding an antigen-binding protein II', and an antigen-binding fragment II', together with the reference antigen-binding fragment I, is capable of forming an antigen-binding protein having the following property: capability of binding to a target, to which the reference antigen-binding protein can bind, at a capacity that is 30% or more of a binding capacity of the reference antigen-binding protein to the target; d) providing a third vector polynucleotide including R6-vector fragment III-R7 in a 5'-to-3' direction; e) providing a fourth vector polynucleotide including R8-vector fragment IV-R5 in a 5'-to-3' direction; f) cleaving, with a restriction endonuclease, the third polynucleotide, the fourth polynucleotide, the third vector polynucleotide and the fourth vector polynucleotide to obtain the cleaved third polynucleotide, the cleaved fourth polynucleotide, the cleaved third vector polynucleotide and the cleaved fourth vector polynucleotide; and g) mixing the cleaved third polynucleotide, the cleaved fourth polynucleotide, the cleaved third vector polynucleotide and the cleaved fourth vector polynucleotide, such that the cleaved third polynucleotide, the cleaved fourth polynucleotide, the cleaved third vector polynucleotide and the cleaved fourth vector polynucleotide can be cyclized by directional linkage to form double-replacement to-be-screened vectors. The structure of the double-replacement to-be-screened vector may be found in FIG. 2. The method further includes: expressing the first to-be-screened vectors, and by comparison with a reference antigen-binding protein (or other positive control antibodies), selecting an antigen-binding protein having the following property, as the functional antigen-binding protein: capability of binding to a target, to which the reference antigen-binding protein can bind, at a capacity that is 30% or more of a binding capacity of the reference antigen-binding protein to the target.

In the present application, the nucleic acid fragment I' may encode an antibody light chain, or a fragment thereof, of a reference antigen-binding protein; the nucleic acid fragment II' may encode an antibody heavy chain or a fragment thereof; the vector fragment III may include a linkage peptide; and the vector fragment IV may be derived from a phage display vector (for example, a pComb3x vector). In some cases, the nucleic acid fragment I' encodes an antibody light chain, and the linkage peptide includes a nucleic acid sequence encoding a signal peptide pelB or a fragment thereof. In some cases, the nucleic acid fragment I' encodes an antibody light-chain variable region. R5 may include a nucleotide sequence as set forth in SEQ ID NO: 1, R6 may include a nucleotide sequence as set forth in SEQ ID NO: 2, R7 may include a nucleotide sequence as set forth in SEQ ID NO: 3, and R8 may include a nucleotide sequence as set forth in SEQ ID NO: 4.

In the present application, the nucleic acid fragment I' may encode an antibody light chain, or a fragment thereof, of a reference antigen-binding protein; the nucleic acid fragment II' may encode a heavy chain or a fragment thereof; and the vector fragment III and/or the vector fragment IV may be derived from a mammalian cell expression vector (for example, a pDGB4 vector). R5 may include a nucleotide sequence as set forth in SEQ ID NO: 7, R6 may include a nucleotide sequence as set forth in SEQ ID NO: 8, R7 may include a nucleotide sequence as set forth in SEQ ID NO: 5, and R8 may include a nucleotide sequence as set forth in SEQ ID NO: 6.

In the present application, the nucleic acid fragment I' may encode an antibody heavy chain, or a fragment thereof, of a reference antigen-binding protein; the nucleic acid fragment II' may encode a light chain, or a fragment thereof, of an antigen-binding protein; the vector fragment IV may include a linkage peptide; and the vector fragment III may be derived from a phage display vector (for example, a pComb3x vector). In some cases, the nucleic acid fragment II' encodes an antibody light chain, and the linkage peptide includes a nucleic acid sequence encoding a signal peptide pelB or a fragment thereof. In some cases, the nucleic acid fragment II' encodes an antibody light-chain variable region. R5 may include a nucleotide sequence as set forth in SEQ ID NO: 3, R6 may include a nucleotide sequence as set forth in SEQ ID NO: 4, R7 may include a nucleotide sequence as set forth in SEQ ID NO: 1, and R8 may include a nucleotide sequence as set forth in SEQ ID NO: 2.

In the present application, the nucleic acid fragment I' may encode an antibody heavy chain or a fragment thereof; the nucleic acid fragment II' may encode a light chain, or a fragment thereof, of a reference antigen-binding protein; and the vector fragment III and/or the vector fragment IV may be derived from a mammalian cell expression vector (for example, a pDGB4 vector). R5 may include a nucleotide sequence as set forth in SEQ ID NO: 5, R6 may include a nucleotide sequence as set forth in SEQ ID NO: 6, R7 may include a nucleotide sequence as set forth in SEQ ID NO: 7, and R8 may include a nucleotide sequence as set forth in SEQ ID NO: 8.

The method of the present application can be used to simultaneously select functional antigen-binding proteins targeting a plurality of targets and a plurality of binding sites of one target. When the method of the present application is used, and when the first to-be-screened vector and/or the second to-be-screened vector is constructed, a plurality of reference antigen-binding proteins targeting the same antigen and binding to different epitopes may be simultaneously used to obtain to-be-screened vectors including a plurality of reference antigen-binding fragments, in order to obtain fragments (nucleic fragments I' and II') encoding the plurality of functional antigen-binding proteins targeting the same antigen but binding to different epitopes. When the method of the present application is used, a plurality of reference antigen-binding proteins targeting different antigens may also be simultaneously used to obtain to-be-screened vectors including a plurality of reference antigen-binding fragments, in order to obtain fragments (nucleic fragments I' and II') encoding the plurality of functional antigen-binding proteins targeting different antigens.

For example, the first to-be-screened vector library may include a first to-be-screened vector containing a plurality of reference nucleic acid fragments II; and the reference nucleic acid fragments I encode fragments of two, three, four or more reference antigen-binding proteins that bind to the same antigen. For example, the second to-be-screened vector library may include a second to-be-screened vector containing a plurality of reference nucleic acid fragments I; and the reference nucleic acid fragments I encode fragments of two, three, four or more reference antigen-binding proteins that bind to the same antigen.

For example, the first to-be-screened vector library may include a first to-be-screened vector containing a plurality of reference nucleic acid fragments II; and the reference nucleic acid fragments II encode fragments of different reference antigen-binding proteins binding to a plurality of (for example, 2, 3, 4, 5, 6, 7 8, 9, 10 or more) antigens. For example, the second to-be-screened vector library may include a second to-be-screened vector containing a plurality of reference nucleic acid fragments I; and the reference nucleic acid fragments I encode fragments of different reference antigen-binding proteins binding to a plurality of (for example, 2, 3, 4, 5, 6, 7 8, 9, 10 or more) antigens.

Therefore, the double-replacement to-be-screened vector library including one or more double-replacement to-be-screened vectors may include more (for example, 2, 3, 4, 5, 6, 7, 8, 9, 10, 11, 12, 13, 14, 15, 16, 17 or more) antigen-binding proteins targeting a plurality of (for example, 2, 3, 4, 5, 6, 7, 8, 9, 10 or more) antigens.

In the present application, for the construction of the moiety of the to-be-screened vector for phage expression, a reference can be made to PCT International Application PCT/CN2020/085706.

In the present application, the linker may include a nucleic acid sequence encoding a fragment of the signal peptide pelB, and the remaining nucleic acid sequences encoding the fragments of the signal peptide pelB may be also located at a recognition site of the polynucleotide. For example, a nucleotide at the 3'-terminus of the nucleic acid sequence encoding the fragment of the signal peptide pelB may be located at the digestion site. For example, after two cleaved polynucleotides are linked, the nucleic acid sequence included in one of the polynucleotides and encoding the fragment of the signal peptide pelB may be linked to the nucleic acid sequence included in the other polynucleotides and encoding the fragment of the signal peptide pelB, thereby forming a nucleic acid sequence encoding a complete signal peptide pelB.

In the present application, the linker may have a length of about 50 bases to about 200 bases. For example, the length of the linker may be about 50 bases to about 200 bases, about 50 bases to about 180 bases, about 50 bases to about 160 bases, about 50 bases to about 140 bases, about 50 bases to about 120 bases, about 50 bases to about 100 bases, about 50 bases to about 90 bases, about 50 bases to about 80 bases, about 50 bases to about 75 bases, about 50 bases to about 70 bases, about 50 bases to about 60 bases.

In the present application, a terminus of any one of R1, R2, R3, R4, R5, R6, R7, R8, R9, R10, R11 and R12 resulting from cleavage by the corresponding restriction endonuclease may include a non-palindromic sequence.

When an endonuclease recognition site (for example, R1, R2, R3, R4, R5, R6, R7, R8, R9, R10, R11 and R12) is selected, sequences that are substantially not included in the nucleic acid fragments (for example, a nucleic acid fragment I, a nucleic acid fragment II, a nucleic acid fragment I', a nucleic acid fragment II', a reference nucleic acid fragment I, and a reference nucleic acid fragment II') can be selected to keep the integrity of the functional regions (for example, a light-chain variable region or a heavy-chain variable region) of the antigen-binding fragments encoded by these sequences, in order to prevent an antibody gene library from being damaged (for example, degraded) during digestion. In addition, a terminus resulting from cleavage of the nucleotide recognition site by the restriction endonuclease may include a non-palindromic sequence, in order to prevent them from self-linkage, thereby reducing non-targeting linkage. In addition, the selection of the endonuclease recognition site may enable the directional linkage of a plurality of fragments, in order to improve the linkage efficiency.

In the present application, two or more (for example, 2, 3, 4, 5, 6, 7, 8, 9, 10, 11 or 12) of R1, R2, R3, R4, R5, R6, R7, R8, R9, R10, R11 and R12 may be recognized and cleaved by the same restriction endonuclease.

In the present application, the restriction endonuclease may be selected from the group consisting of *SfiI*, *BsmBI* and *Esp3I.* In the present application, *BsmBI* and *Esp3I* may be isozymes and may recognize the same restriction endonuclease recognition site.

For example, *SfiI* may recognize a sequence consisting of 13 bases (from 5' to 3'), namely, GGCCNNNN/NGGCC (SEQ ID NO: 9), which may be digested to form an overhang sequence (for example, a single-chain sequence including 3 bases) at the 3'-terminus, with N capable of representing any one of four bases including GATC. Therefore, there are 64 different sequences that can be recognized by *SfiI.*

For example, *BsmBI* and *Esp3I* may recognize a sequence consisting of 12 bases (from 5' to 3'), namely, CGTCTCN/NNNNN (SEQ ID NO: 10), which may be digested to form an overhang sequence (for example, a single-chain sequence including 4 bases) at the 5'-terminus, with N capable of representing any one of four bases including GATC. Therefore, there are 264 different sequences that can be recognized by *BsmBI* and *Esp3I.*

In the present application, in order to construct the vector of the present application, the phage display vector or mammalian cell display vector (for example, pDGB4 and/or pComb3x vector(s)) may be engineered/modified. For example, one or more endonuclease recognition sites in the vector may be removed by means of site-directed mutation. In some cases, it is also possible to add one or more endonuclease recognition sites in the vector by means of site-directed mutation.

For example, the pComb3x vector may be modified to adapt to the object of the present application. For example, the pComb3x vector may include one *SfiI* recognition site at the 5'-terminus and one *SfiI* recognition site at the 3'-terminus. During the modification, the *SfiI* recognition site at the 3'-terminus in the pComb3x vector may be removed by means of site-directed mutation. In some cases, the site-directed mutation may not affect the in-frame expression of the protein (for example, the antibody heavy-chain fragment and/or the antibody light-chain fragment) in the vector. For example, the mutation may be nonsense mutation, for example, the mutation that only changes a base sequence but does not change an amino acid sequence.

In some cases, when mammalian cell expression is used for the first to-be-screened vector, the second to-be-screened vector and/or the double-replacement to-be-screened vector, the vector fragment (for example, a vector fragment I, a vector fragment II, a vector fragment III, a vector fragment IV, a vector fragment V and a vector fragment VI) may be from any vector capable of expressing a gene of interest. For example, the vector fragment may be a fragment from a display vector pDGB4 (for pDGB4, see Ivan Zhou, et al., "Four-way ligation for construction of a mammalian cell-based fµLl-length antibody display library", Acta Biochim Biophys Sin 2011, 43: 232-238).

The vector fragment (for example, a vector fragment I, a vector fragment II, a vector fragment III, a vector fragment IV, a vector fragment V and a vector fragment VI) of the present application may include nucleotide sequence having specific functions, including but not limited to, a promoter, an enhancer, a signal peptide, and a screening marker (for example, which may include an enzyme recognition site, a resistance gene, a reporter gene, and a screening gene), which may be adjusted (insertion/substitution and/or deletion of the above nucleotide sequences having the specific functions) in the vector fragment by those skilled in the art according to the desired function. In some cases, the vector fragment may be adjusted under different conditions to obtain different nucleotide sequences.

In the present application, the vector may include or encode one or more appropriate markers (for example, markers for purifying/recognizing/screening), which may include, for example, His tag, Flag Tag, fluorescent proteins, selective antibiotics, and/or avidins, or the like.

The vector fragment (for example, a vector fragment I, a vector fragment II, a vector fragment III, a vector fragment IV, a vector fragment V and/or a vector fragment VI) of a desired length or type can be selected according to the length or property of an antigen-binding fragment to be expressed and the length or property of a digestion site.

In the present application, the nucleic acid fragment I and the nucleic acid fragment II may be obtained from a sample material. In the present application, the sample material may include a material derived from a peripheral blood lymphocyte sample. In the present application, the peripheral blood lymphocyte may be a human peripheral blood lymphocyte. In the present application, the sample material may also be derived from other arbitrary tissues and/or cells, and is not limited to the peripheral blood lymphocyte sample.

For example, the nucleic acid fragment I and the nucleic acid fragment II may be obtained from the constructed library of antibody light chains or fragments thereof or the constructed library of antibody heavy chains or fragments thereof in the prior art. The method includes adding the digestion sites of the present application to both termini of one or more antibody heavy chains or fragments thereof, or one or more antibody light chains or fragments thereof.

In the present application, the binding capacity of the expression product(s) of the first to-be-screened vector, the second to-be-screened vector and/or the double-replacement to-be-screened vector to the target may be detected using various methods. The binding capacity of the expression products of the to-be-screened vectors to a reference antigen-binding protein or other positive controls may be compared by quantitative or nonquantitative method.

In one aspect, for example, the target-binding activity of the antigen-binding protein of the present application may be tested by known methods such as enzyme-linked immunosorbent assay (ELISA), immunoblotting (for example, Western blotting), flow cytometry (for example, FACS), immunohistochemistry, and immunofluorescence. In some cases, the binding affinity may be determined by surface plasmon resonance (SPR), enzyme-linked immunoassay (ELISA), binding antigen precipitation, equilibrium dialysis, or biofilm interferometry (BLI). In some cases, the PD-1 binding affinity and KD value of a PD-1 antigen-binding protein may be determined by biofilm interferometry (BLI). For example, ELISA may be used for determination. For example, under a condition allowing binding of the antigen-binding protein and/or the reference antigen-binding protein to the target, the expression product(s) of the first to-be-screened vector, the second to-be-screened vector and/or the double-replacement to-be-screened vector, and/or the reference antigen-binding protein, are/is allowed to contact the target (for example, antigen), to detect the formation of a complex between the expression product(s) of the first to-be-screened vector, the second to-be-screened vector and/or the double-replacement to-be-screened vector and the target, or detect the formation of a complex between reference antigen-binding protein and the target.

For the quantitative method, the target-binding capacity may include an EC₅₀ value for target binding, a KD value for target binding, an OD value of a complex resulting from target binding, and/or absorbance of the complex resulting from target binding. When the EC₅₀ value, KD value, OD value of the complex, and/or absorbance of the complex resulting from target binding of the first to-be-screened vector, the second to-be-screened vector and/or the double-replacement to-be-screened vector are 30% or more (for example, 35%, 40%, 45%, 50%, 55%, 60%, 65%, 70%, 75%, 80%, 85%, 90%, 95%, 99%, 100%, 110% or 120%, or more) of the EC₅₀ value, KD value, OD value of the complex, and/or absorbance of the complex resulting from target binding of the reference antibody, the expression product(s) may be considered as being capable of specifically binding to the target. Alternatively, when the EC₅₀ value, KD value, OD value of the complex, and/or absorbance of the complex resulting from target binding of the first to-be-screened vector, the second to-be-screened vector and/or the double-replacement to-be-screened vector are 2 times or more (for example, 2.5 times, 3 times, or 3.5 times or more) those of the negative controls without the expression product(s) of the first to-be-screened vector, the second to-be-screened vector, the double-replacement to-be-screened vector, and/or the reference antigen-binding protein, the expression product(s) may also be considered as being capable of specifically binding to the target.

In another aspect, the present application provides a method for preparing the functional antigen-binding protein, including the use of the first to-be-screened vector, the second to-be-screened vector, and/or the double-replacement to-be-screened vector.

In another aspect, the present application provides a functional antigen-binding protein obtained by means of the method of the present application.

Not wishing to be bound by any theory, the following examples are merely to explain the method of the present application and the functional antigen-binding protein prepared by the method of the present application, and are not intended to limit the scope of the invention of the present application.

### EXAMPLES

### Example 1: Preparation of TG1 Bacteria Carrying Phage Display Vectors of Fabs of 16 Reference Antibodies

### 1.1 Synthesis of Genes of Full-length Light- and Heavy-chain Variable Regions of 16 Reference Antibodies

The amino acid sequences of full-length light- and heavy-chain variable regions of the following 16 reference antibodies were obtained through literature search: Secukinumab, Selicrelumab, APX-005M, Tafasitamab, Ublituximab, Epratuzumab, Relatlimab, Leramilimab, Coblimab, Tiraglumab, Vibostolimab, Etigilimab, Amatuximab, TRX-518, Bleselumab, and Iscalimab.

For example, the amino acid sequences of the full-length light and heavy chains of Selicrelumab were obtained through literature search (see related sequence information in WO2018220100). Here, the amino acid sequence of the full-length light chain of Selicrelumab was as set forth in SEQ ID NO. 11, and the amino acid sequence of the full-length heavy chain of Selicrelumab was as set forth in SEQ ID NO. 12.

The amino acid sequences of the full-length light- and heavy-chain variable regions of Selicrelumab were converted into base sequences expressible in Escherichia coli, where base sequences matching the phage display vectors and including appropriate digestion sequences and (or) signal peptide sequences were added to both termini of these amino acid sequences, which were then synthesized into a gene that was then inserted into a pUC57 vector (synthesized by Genewiz Biotech Co., Ltd.).

The synthesized nucleotide sequence including the full-length light chain was as set forth in SEQ ID NO. 13, and the synthesized nucleotide sequence including the heavy-chain variable region was as set forth in SEQ ID NO. 14.

The amino acid sequences of the full-length light- and heavy-chain variable regions of the 16 reference antibodies were converted into base sequences expressible in Escherichia coli, where base sequences matching the phage display vectors and including appropriate digestion sequences and (or) signal peptide sequences were added to both termini of these amino acid sequences, which were then synthesized into genes that were then inserted into pUC57 vectors (synthesized by Genewiz Biotech Co., Ltd.).

### 1.2 Construction of Phage Display Vectors of Fabs of 16 Reference Antibodies

The pUC57 vector including the synthesized base sequences of 16 full-length light chains were digested with SfiI to obtain a fragment of 16 full-length light chains, i.e., the first polynucleotide in FIG. 1.

The pUC57 vector including the synthesized base sequences of 16 heavy-chain variable regions were digested with SfiI+Esp3I to obtain a fragment of 16 heavy-chain variable regions, i.e., the second polynucleotides in FIG. 1.

The fragment of 16 full-length light chains (i.e., the first polynucleotide in FIG. 1 of the present application) and the fragment of 16 heavy-chain variable regions (i.e., the second polynucleotide in FIG. 1 of the present application) were subjected to 16 four-fragment linkages in terms of the fragment of light chains corresponding to the reference antibodies, the fragment of heavy-chain variable regions corresponding to the reference antibodies, and the first and second polynucleotides indicated in FIG. 1; the cloned sequences were sequenced and analyzed to obtain the phage display vectors for Fabs of 16 positive reference antibodies. The description and preparation of the first and second vector polynucleotides could be found in relevant steps in Patent Application WO2020216191A1 for library construction.

For example, the obtained full-length light chain fragment (first polynucleotide) of Selicrelumab and the obtained heavy-chain variable region fragment (second polynucleotide) of Selicrelumab were subjected to four-fragment linkage with the first and second vector polynucleotides indicated in FIG. 1; and the cloned base sequences were sequenced and analyzed to obtain the phage display vector of the positive benchmark antibody Selicrelumab-Fab. The description and preparation of the first and second vector polynucleotides could be found in relevant steps in WO2020216191A1.

Specifically, the above first vector polynucleotide was recorded as in Example 1.6 of WO2020216191A1: by taking the linkage component plasmid or the linker storage vector in Example 1.4.4 as a template, a 0.8 kb linker-containing fragment was amplified with the forward primer (SEQ ID NO: 79 in WO2020216191A1) and reverse primer (SEQ ID NO: 80 in WO2020216191A1) of the linker, and then the PCR product of the 0.8 kb was digested with the restriction endonucleases R3 and R4, purified for recovery by gel electrophoresis (with a small fragment gel recovery kit, purchased from LifeFeng Biotech, Cat #DK402) to obtain a 72pb linker fragment.

The above second vector polynucleotide was recorded as in Example 1.8 of WO2020216191A1: the display vector DDB-R1R2R5R6 prepared in Example 1.7 was digested with the restriction endonuclease R7 and the restriction endonuclease R8 to obtain a 3.6 kb display vector fragment.

### 1.3 Preparation of TG1 Bacteria Carrying Phage Display Vectors for Fabs of 16 Reference Antibodies

The phage display vectors for Fabs of 16 reference antibodies were introduced into TG1 electrocompetent cells (Lucigen, Cat#: 60502) to obtain TG1 bacteria carrying the phage display vector for Fab of each reference antibody.

For example, the phage display vector of the positive benchmark antibody Selicrelumab-Fab was introduced into TG1 electrocompetent cells to obtain the TG1 bacteria carrying the phage display vector of the benchmark antibody Selicrelumab-Fab.

### Example 2 Construction of Light-chain Replacement Gene Library, Bacteria Library and Phage Library for Multiple Reference Antibodies

1. A full human x-light-chain and λ-light-chain sublibrary was digested with SfiI to prepare insertion fragments for a κ-light-chain library and a λ-light-chain library.
2. The obtained insertion fragments (first polynucleotides) for the κ-light-chain library and the λ-light-chain library, the heavy-chain variable region fragments of 16 reference antibodies (second polynucleotides), and the first and second polynucleotides indicated in FIG. 1 were subjected to 4-fragment linkage to construct a light-chain replacement library. The four fragments in a total amount of 2345 ng were mixed at equal molecular number ratio of 1:1:1:1 and linked at 20°C overnight (15 h).
3. A linkage product was purified to obtain 1062 ng of linkage products of the light-chain replacement library (gene library).
4. Transformed three tubes of TG1 electrocompetent cells with a total volume of 6000 µl of bacterial solution, and cultured for 60 min at 37°C/250 rpm, and then, 10 µl of the bacterial solution was subjected to titration for the transformation efficiency of the linkage. 10x dilution was performed 8 times, and for each dilution, 5 µl of the bacterial solution was spread on a dish and cultured at 32°C overnight.
5. The remaining bacterial solution was diluted with 220 ml of 2YT-Amp-2% glucose culture solution, cultured for 3 h at 37°C/250 rpm, and then measured to obtain OD₆₀₀=0.6 (bacteria library).
6. 50 ml of the bacterial solution of the bacteria library from step 7 was transferred to a 150 ml shake flask, continuously cultured for 4 h at 37°C/250 rpm, and centrifuged to collect bacteria; the bacteria were suspended with 4 ml of 2TY-Amp-7% DMSO, subpackaged in two tubes, and cryopreserved at -80°C.
7. 3 ml of helper phages (2.9×10¹³/ml) were added to the remaining bacterial solution of the bacteria library from step 7, stood for 40 min at 37°C, cultured for 40 min at 37°C/250 rpm,
8. and centrifuged for 15 min at 3500 G to collect bacteria infected by the helper phages, and the supernatant was discarded. The bacteria were suspended with 500 ml of 2YT-Amp-Kan culture solution, and cultured overnight at 30°C/250 rpm.
9. Next day, the capacity of the light-chain replacement bacteria library was analyzed and calculated as 1.2×10⁹ according to the results of titration and dish spreading from step 4. The bacteria cultured overnight were centrifuged and precipitated; 500 ml of supernatant was mixed with 125 ml of 20% PEG-5M NaCl, treated in an ice bath for 2 h, and then centrifuged to collect a light-chain replacement phage library.
10. The light-chain replacement phage library resulting from centrifugation and precipitation was suspended with 5 ml of PBS, and centrifuged for 5 min at 4°C/18000 g on a benchtop high speed centrifuge; the supernatant was collected to detect OD₂₈₀; and the concentration of the light-chain replacement phage library was calculated as follows: with OD₂₈₀=13.7, the phage concentration was 3.2×10¹³/ml.
11. The light-chain replacement phage library was cryopreserved as follows: 5 ml of phage library plus 2 ml of 80% autoclaved glycerine plus 0.5 ml of PBS, in a total of 7.5 ml, were mixed uniformly, subpackaged in 0.5 ml/1×10¹³/tubes, and cryopreserved at -80°C _{°}

### Example 3 Construction of Heavy-chain Replacement Gene Library, Bacteria Library and Phage Library for Multiple Reference Antibodies

1. A full human heavy-chain variable region (Vh) sublibrary was digested with SfiI and Esp3I to prepare insertion fragments for a Vh library.
2. Referring to the steps described in Example 1, the obtained insertion fragments (first polynucleotides) for light chains of 16 reference antibodies, the obtained insertion fragments (second polynucleotides) for the Vh library, and the first and second vector polynucleotides indicated in FIG. 1 were subjected to 4-fragment linkage to construct a light-chain replacement library. The four fragments in a total amount of 2345 ng were mixed at equal molecular number ratio of 1:1:1:1 and linked at 20°C overnight (15 h).
3. A linkage product was purified to obtain 1134 ng of linkage products of the heavy-chain replacement library (gene library).
4. Transformed three tubes of TG1 electrocompetent cells with a total volume of 6000 µl of bacterial solution, and cultured for 60 min at 37°C/250 rpm, and then, 10 µl of the bacterial solution was subjected to titration for the transformation efficiency of the linkage. 10× dilution was performed 8 times, and for each dilution, 5 µl of the bacterial solution was spread on a dish and cultured at 32°C overnight.
5. The remaining bacterial solution was diluted with 220 ml of 2YT-Amp-2% glucose culture solution, cultured for 3 h at 37°C/250 rpm, and then measured to obtain OD₆₀₀=0.6 (bacteria library).
6. 50 ml of the bacterial solution of the bacteria library from step 7 was transferred to a 150 ml shake flask, continuously cultured for 4 h at 37°C/250 rpm, and centrifuged to collect bacteria; the bacteria were suspended with 4 ml of 2TY-Amp-7% DMSO, subpackaged in two tubes, and cryopreserved at -80°C.
7. 3 ml of helper phages (2.9×10¹³/ml) were added to the remaining bacterial solution of the bacteria library from step 7, stood for 40 min at 37°C, cultured for 40 min at 37°C/250 rpm,
8. and centrifuged for 15 min at 3500 G to collect bacteria infected by the helper phages, and the supernatant was discarded. The bacteria were suspended with 500 ml of 2YT-Amp-Kan culture solution, and cultured overnight at 30°C/250 rpm.
9. Next day, the capacity of the heavy-chain replacement bacteria library was analyzed and calculated as 1.2×10⁹ according to the results of titration and dish spreading from step 4. The bacteria cultured overnight from step 10 were centrifuged and precipitated; 500 ml of supernatant was mixed with 125 ml of 20% PEG-5M NaCl, treated in an ice bath for 2 h, and then centrifuged to collect a heavy-chain replacement phage library.
10. The light-chain replacement phage library resulting from centrifugation and precipitation was suspended with 5 ml of PBS, and centrifuged for 5 min at 4°C and 18000 g on a benchtop high speed centrifuge; the supernatant was collected to detect OD₂₈₀; and the concentration of the heavy-chain replacement phage library was calculated as follows: with OD₂₈₀=20.3, the phage concentration was 4.7×10¹³/ml.
11. The heavy-chain replacement phage library was cryopreserved as follows: 5 ml of phage library plus 2 ml of 80% autoclaved glycerine plus 0.5 ml of PBS, in a total of 7.5 ml, were mixed uniformly, subpackaged in 0.5 ml/1×10¹³/tubes, and cryopreserved at -80°C.

### Example 4: Preparation of Tg1 Bacteria Carrying Phage Display Vector of Fab of Reference Antibody Her3/Patritumab and Construction of Light- and Heavy-chain Replacement Gene Libraries, Bacteria Library and Phage Library for Single Reference Antibody (Patritumab)

1. Referring to the procedures of Example 1, Tg1 bacteria carrying a phage display vector of Fab of a reference antibody (Patritumab) was prepared. The CDR related sequence of Patritumab could be found in CN 102633881B. The light- and heavy-chain sequences of Patritumab could be found in US 7705130B2.
2. Referring to the procedures for constructing the light- and heavy-chain replacement libraries of multiple reference antibodies, the light-chain replacement library and the heavy-chain replacement library were constructed for the single reference antibody Patritumab.
3. With two linkage systems for the light-chain replacement library and the heavy-chain replacement library, the total quantity of linkage fragments was 1173 ng for each system; linkage was conducted for 6 h; linkage products were purified; and 550 ng was obtained for each system.
4. 250 ng from each system was used to transform one tube of TG1 electrocompetent cells. The volume of converted bacterial solution was 2 ml for each system, with 10 µl for titration and the remaining for spreading a large dish and a medium dish. Overnight culturing was performed at 32°C.
5. On the second day, the capacity of the bacteria library was calculated according to titration results, with 2.4 x10e8 for the heavy-chain replacement library and 4.4 × 10e8 for the light-chain replacement library. All bacterial colonies were collected from the two bacteria libraries respectively; 50 ml of culture solution (2YT-0.2%glucose-Amp) was formulated for each library; and a proper amount of bacterial solution was added to each culture solution, of which OD₆₀₀ was determined as about 0.12.
6. Each resultant was cultured for 50 min at 37°C/250 rpm, and OD₆₀₀ was determined as about 0.25. 250 µl of helper phages were added to each resultant (OD₂₈₀=13). After uniform mixing, each resulting mixture was subjected to static culturing for 30 min at 37°C, and for 30 min at 37°C/250 rpm.
7. Each mixture was centrifuged to collect bacteria, which were then resuspended with 100 ml of culture solution (2YT-Amp-Kana) and cultured for 6 h at 30°C/250 rpm (3 pm-9 pm). The bacteria were centrifuged and precipitated to collect a supernatant respectively; and 25 ml of 5xPEG-NaCl was added to each supernatant, which was then mixed uniformly and subjected to overnight precipitation at 4°C for phages.
8. On the third day, the light-chain replacement phage library and the heavy-chain replacement phage library were collected by centrifugation. Each phage library was suspended with 2 ml of PBS, subpackaged in 4 tubes and cryopreserved at -80°C.

### Example 5 Construction of Heavy-chain Replacement Gene Library, Bacteria Library and Phage Library for Single Reference Antibody (CD40/Secukinumab)

1. Referring to the procedures (Examples 3) for constructing the heavy-chain replacement library for multiple reference antibodies, the heavy-chain replacement library was constructed for the single reference antibody Secukinumab.
2. Referring to the process in Example 1, the obtained insertion fragment (first polynucleotide) for the light chain of Selicrelumab, the full human insertion fragment (second polynucleotide) for the Vh library, and the first and second vector polynucleotides indicated in FIG. 1 were subjected to four-fragment linkage to construct a heavy-chain replacement library. The four fragments in a total amount of 1175 ng were mixed at equal molecular number ratio of 1:1:1:1 and linked at 20°C overnight (15 h).
3. A linkage product was purified to obtain 513 ng of linkage products of the heavy-chain replacement library (gene library).
4. Transformed One tube of TG1 electrocompetent cells with 300 ng of the linkage product into a bacterial solution with a total volume of 2000 µl, and cultured for 60 min at 37°C and 250 rpm, and then, 10 µl of the bacterial solution was subjected to titration for the transformation efficiency of the linkage. 10× dilution was performed 8 times, and for each dilution, 5 µl of the bacterial solution was spread on a dish and cultured at 32°C overnight.
5. The remaining bacterial solution was equally spread in two large dishes and cultured at 32°C overnight.
6. Next day, the capacity of the heavy-chain replacement library was analyzed and calculated as 3.2×10⁸ according to the result of titration from step 4. All bacterial colonies were collected from the two large dishes cultured overnight in step 5, and the total volume of the bacterial solution was 15 ml. The bacterial solution was determined with OD₆₀₀=20. DMSO was added to 3 ml of the bacterial solution (with the final concentration of 7%), subpackaged in two tubes and cryopreserved at -80°C (the bacteria library).
7. 200 µl of the cryopreserved bacterial solution collected in step 6 was added to 50 ml of 2YT-Amp-0.2% glucose culture solution, and OD₆₀₀ was determined as 0.109; and the resulting mixture was cultured for 60 min at 37°C and 250 rpm, and OD₆₀₀ was determined as 0.247.
8. 250 µl of helper phages (OD₂₈₀=13) were added to the mixture, mixed uniformly, stood for 30 min at 37°C, and cultured for 30 min at 37°C and 250 rpm,
9. The bacteria infected by the helper phages was collected by centrifugation, suspended in 100ml of 2YT-Amp-Kan culture solution, and cultured for 8 h at 30°C and 250 rpm. The bacteria were centrifuged and precipitated to collect a supernatant, which was then uniformly mixed with 25 ml of 5x PEG-NaCl and subjected to precipitation overnight at 4°C for phages.
10. Next day, the phage library from step 9 was centrifuged and precipitated, and the phage precipitate was suspended with 2 ml of PBS to obtain the phage library. OD₂₈₀ was determined as 13, and the phage concentration was determined as 3×10¹³/ml; and 0.2 ml of heavy-chain replacement phage library was used for the first round of screening.
11. The heavy-chain replacement phage library was cryopreserved as follows: 0.6 ml of autoclaved 80% glycerine solution was added till the final glycerine solution was 20%, the resultant was mixed uniformly, subpackaged in 4 tubes (0.6 ml per tube) and cryopreserved at -80°C.

### Example 6: Screening of Light-chain Replacement Positive Clones of CD40 Antigen-specific Selicrelumab

### Preparation of antigens:

Biotin-labeled CD40 antigens (AcroBio, Cat#: CD0-H82E8) and Biotin-label-free CD40 antigens (AcroBio, Cat#: CD0-H5253) were purchased; their freeze-dried powder was dissolved according to the instructions, the biotin-labeled antigens were subpackaged at 5µg per tube, the biotin-free antigens were subpackaged at 10 µg per tube, and all the tubes were preserved at -80°C.

### First round of screening:

1. One tube of light-chain replacement phage library (with the phage concentration of 1×10¹³/0.5ml, Example 2) of multiple reference antibodies, which was cryopreserved at -80°C, was thawed in an ice bath and mixed with 100 µl of MPBST (1X PBS containing 12% milk and 0.05% Tween), with the final volume of 0.6 ml and the final milk concentration of 2%.
2. 60 µl of cleaned and resuspended magnetic beads (Invitrogen, Cat#: 11206D) were added and rotationally shaken for 30 min at room temperature to absorb non-specific phages capable of binding to the magnetic beads.
3. A magnetic rack (Invitrogen Cat#: 12321D) was used to absorb and discard the magnetic beads; the phage library solution was transferred to a new 2 ml EP tube blocked with 2% MPBST.
4. 5 µg of biotin-labeled antigens were mixed with the light-chain replacement phage library (with the final antigen concentration of 5 µg/0.6ml) and rotationally shaken for 2h at room temperature, whereby phages displaying antigen-specific Fabs bound to the biotin-labeled antigens.
5. 60 µl of the cleaned and suspended magnetic beads were added to the phage library solution, and rotationally shaken for 20 min at room temperature, such that antigen-specific phases were captured by the binding of avidins on the surfaces of the magnetic beads to the biotins, thereby forming a magnetic bead-avidin-biotin-antigen-Fab antibody fragment complex.
6. The magnetic beads carrying the antigen-specific Fab complex as formed in step 5 were collected by the magnetic rack.
7. The magnetic beads were washed 4 times with 1xPBST, and then 4 times with 1xPBS.
8. The magnetic beads were suspended with 300 µl of glycine solution of pH 2.2, and rotationally shaken for 10 min at room temperature, and the phages displaying the antigen-specific Fabs were eluted.
9. The magnetic beads were collected by a magnetic rack; the eluted supernatant containing no magnetic beads was transferred to a new 1.5 ml EP tube, and neutralized to pH 7.0 with 110 µl of Tris buffer of pH 8.0.
10. The magnetic beads were resuspended with 100 µl of glycine solution of pH 2.2, and rotationally shaken for 5 min, and the phages displaying the antigen-specific Fabs were eluted.
11. The magnetic beads were collected by a magnetic rack; the eluted supernatant containing no magnetic beads was transferred to a new 1.5 ml EP tube, and neutralized to pH 7.0 with 35 µl of Tris buffer of pH 8.0.
12. The neutralized eluted phage solutions obtained in steps 9 and 11 were combined (about 540 µl) and preserved on ice for later use.
13. 40 ml of TG1 bacteria were precultured, and OD₆₀₀ was monitored to reach 0.2-0.3.
14. 3 ml of TG1 bacterial solution was mixed with 400 µl of the neutralized eluted phage solution, and stood for 30 min at 37°C.
15. 10 µl of the bacterial solution infected by the phage library was diluted 10 folds eight times using the 2YT culture solution; for each dilution, 5 µl of the bacterial solution was spread on a 2YT-Amp flat dish and cultured at 32°C overnight; and the titration for screening efficiency was conducted.
16. The remaining bacterial solution was spread on two large square flat dishes (Thermo Cat#: 8-1030-051), and cultured overnight at 32°C.
17. 140 µl of the remaining neutralized eluted phage solution was preserved at 4°C.
18. Next day, the phage output from the first round of screening of the light-chain replacement phage library was analyzed and calculated to be 8.8 × 10⁸.

Packaging, amplification, precipitation and collection of phage library obtained from first round of screening:
1. the colonies (with the final bacterial solution volume of 10 ml) on the two large dishes from the first round of output were collected, and 4 ml of the colonies was cryopreserved in two tubes (2ml /7%DMSO/tube).
2. 20 µl of the collected bacterial solution was diluted 50 folds using 2YT, OD₆₀₀ was determined to be 0.7, and OD₆₀₀ of the original bacterial solution was 35.
3. 60 ml of 2YT-Amp-2% Glocose culture solution was formulated; 200 µl of bacteria library solution with OD₆₀₀=0.7 was added; the resulting mixture was cultured for 1 h at 37°C and 250 rpm; and OD₆₀₀ was measured to be about 0.35.
4. 0.5 ml of helper phages (OD₂₈₀=13) were added and mixed with the bacteria library solution, with MOI ranging between 500 and 1000. The resulting mixture stood for 30 min at 37°C, and was cultured for 30 min at 37°C and 250 rpm.
5. The bacteria was collected by centrifugation, resuspended with 100 ml of 2YT-Amp-Kan culture solution, and cultured overnight at 30°C and 250 rpm; and the phages output from the first round were packaged and amplified.
6. Next day, the mixture was centrifuged for 15 min at 4°C and 9000 rpm (Beckman, JA-10); the supernatant was uniformly mixed with 25 ml of 20% PEG-5M NaCl, and treated in an ice bath for 2 h.
7. The resultant was centrifuged for 15 min at 4°C and 9000 rpm (Beckman, JA-10); the supernatant was discarded; the resultant was centrifuged for another 5 min at 4°C; and the phage precipitates were gathered at the bottom of the centrifuge tube, and the residual supernatant was aspirated and discarded by a tip.
8. The phage library was suspended with 2ml of PBS, and centrifuged for 5 min at 4°C and 18000 g on a benchtop high speed centrifuge; the supernatant was collected; OD₂₈₀ was measured to be 16; and the phage concentration was calculated to be 3.73 × 10¹³/ml.
9. 0.5 ml of the phage library was used for a second round of screening.
10. The remaining phage library was uniformly mixed with autoclaved 80% glycerine solution (with the final glycerine concentration of 20%) accounting for 1/3 of the remaining phage library; and the resulting mixture was subpackaged in three tubes at 0.5 ml per tube for each library, and cryopreserved at -80°C.

### Second to Fifth rounds of screening:

1. 0.5 ml of the phage library obtained from the first round of screening was amplified and collected for the second round of screening. The screening process was the same as that of the first round of screening, but the final antigen concentration was 1 µg/0.6ml during screening to obtain 540 µl of eluted and neutralized phage solution.
2. 0.5 ml of the eluted and neutralized phage solution from the second round was used for the third round of screening without amplification. The screening process was the same as that of the first round of screening. During screening, the final antigen concentration was 1 µg/0.6ml to obtain 270 µl of eluted and neutralized phage solution.
3. 200 µl of the eluted and neutralized phage solution from the third round was used to infect 1 ml of TG1 bacteria for 30 min at 37°C; 10 µl of the resultant was used for titration and subjected to 8 gradients of dilutions: for each dilution, 5 µl of the bacterial solution was spread on a 2YT-Amp flat dish, cultured overnight at 32°C and then titrated for screening efficiency. For the remaining bacterial solution, 750 µl was spread on a large dish, 250 µl was spread on a large dish, and both were cultured overnight at 32°C.
4. Next day, the total counts of light-chain replacement phages included in 200 µl of the eluted and neutralized phage solution from the third round were calculated as: 2.6 × 10⁵.
5. The phage library obtained from the third round of screening was packaged, amplified, precipitated and collected, and the process was the same as those in the first round.
6. 0.5 ml of the phage library obtained from the third round of screening was amplified and collected for the fourth and fifth rounds of screening. The screening process was the same as that in the second and third rounds of screening, and the final antigen concentration was 1 µg/0.6ml during screening.
7. 200 µl of the eluted and neutralized phage solution from the fifth round of elution was used to infect 1 ml of TG1 bacteria for 30 min at 37°C; 10 µl of the bacterial solution was titrated; and for the remaining bacterial solution, 750 µl was spread on a large dish, 250 µl was spread on a large dish, and both were cultured overnight at 32°C.
8. Next day, the total counts of light-chain replacement phages in 200 µl of the phage solution from the fifth round were calculated as 1.7 × 10⁶.

Inoculation in deep-well plate and ITPG induction of Fab expression:
1. an inoculation culture solution 2YT-Amp-0.2% Glucose was formulated, and 40 ml of the inoculation culture solution was added to each piece of deep-well plate, at 400 µl/ well.
2. The colonies from the fifth round of screening was inoculated (to one piece of deep-well plate), and the deep-well plate was covered with a breathable film and cultured for 6 h at 37°C and 250 rpm.
3. An IPTG induction culture solution 2YT-Amp-2mM-IPTG was formulated, and 40 ml of the induction culture solution was used for each piece of deep-well plate; and the induction culture solution (400 µl/well) was added to the deep-well plate that was inoculated and cultured for 6 h (with the final IPTG concentration of 1 mM).
4. The culture condition was adjusted to 30°C and 250 rpm, under which the deep-well plate was induced and cultured overnight.
5. A CD40-specific antigen coating solution (9.5ml of ddHzO + 0.5ml of 20x coating solution + 10 µg of biotin-label-free CD40 antigens) was formulated and coated one piece of ELISA plate (at 100 µl/well) overnight at 4°C.

ELISA analysis and screening of CD40 antigen-specific light-chain replacement positive clones:
1. 3% MPBST (10ml/ELISA plate) and PBST (200 µl/each plate washing/well) were formulated.
2. The ELISA plate coated overnight at 4°C was inclined to discard the coating solution, and washed three times with PBST.
3. 3% MPBST (100 µl/well) was added, the plate was covered with a nonbreathable film and blocked for 60 min at 37°C.
4. The plate was inclined to discard the blocking solution and washed three times with PBST.
5. The bacterial solution from the overnight induced deep-well plate was uniformly mixed and added to corresponding wells of the ELISA plate at 100 µl/well, and the plate was covered with a non-breathable film and incubated for 60 min at 37°C.
6. The plate was inclined to discard the bacterial solution and washed three times with PBST.
7. A secondary antibody solution was formulated as follows: 5 ml of PBS was added to 5 µl of Anti-flag-HRP solution (GenScript, Cat# A0148-100, dissolved with ddHzO, 0.5 µg/µl). The formulated secondary antibody solution was formulated and added to the ELISA plate at 50 µl/well; and the plate was covered with a non-breathable film and incubated for 45 min at 37°C.
8. The plate was inclined to discard the second antibody solution and washed three times with PBST.
9. A color developing solution (ABTS, Invitrogen, Cat# MD21704) was added at 50 µl/well for color development for 30 min at room temperature.
10. A stopping solution (0.1 M critic acid) was added at 50 µl/well and mixed uniformly, and the OD₄₀₅ value was read.
11. Clones with OD₄₀₅ greater than 1.2 were sequenced and analyzed to obtain 43 Selicrelumab light-chain replacement positive clones, which were cryopreserved at -80°C.

### Example 7: Screening of Heavy-chain Replacement Positive Clones of CD40 Antigen-specific Selicrelumab

### First round of screening:

200 µl of (with the phage concentration of 3×10¹³/ml, Example 5) of freshly prepared heavy-chain replacement phage library of single reference antibody without glycerine added was mixed with 200 µl of MPBST (1X PBS containing 4% Milk and 0.05% Tween), with a final volume of 0.4 ml and a final Milk concentration of 2%.

The heavy-chain replacement phages were screened with liquid-phase magnetic beads, and the screening process was the same as that for the first round of screening of the light-chain replacement phage library. The antigen concentration was 1 µg/400µl, and the volume of the final eluted and neutralized phage solution was 540 µl. 400 µl of the eluted and neutralized solution was used to infect 1 ml of TG1 bacteria; 10 µl of the resulting bacterial solution was titrated, 1 ml of the bacterial solution was spread on a large dish, and the remaining bacterial solution was spread on another large dish, and both were cultured overnight at 32°C.

The phage output from the first round of screening of the heavy-chain replacement phage library was 8.5 × 10⁵.

The heavy-chain replacement phage library obtained from the first round of screening was packaged, amplified, precipitated, and collected as follows:
the basic process was the same as the process of packaging, amplifying, precipitating and collecting the light-chain replacement phage library obtained from the first round of screening. It was briefly described as follows:
all colonies (6.3x10e5) were collected from the large dish on which 1 ml of the bacterial solution was spread, with the total volume of 8 ml, and the colonies were cryopreserved in two tubes. 140 µl of the collected bacterial solution and 50 ml of the culture solution (2YT-A-G) were uniformly mixed (with measured OD₆₀₀=0.114) and cultured for 60 min at 37°C (with measured OD₆₀₀=0.234); 250 µl of M13KO7 helper phages (OD₂₈₀=13) were added, uniformly mixed, stood for 30 min at 37°C and for another 30 min at 37°C and 250 rpm. The bacteria were collected by centrifugation, resuspended in 100ml of 2YT-Amp-Kan culture solution, and cultured for 8 h at 30°C. The supernatant of the culture solution was collected by centrifugation, uniformly mixed with 25 ml of 5x PEG-NaCl and subjected to precipitation overnight at 4°C for phages. Next day, the phage library was collected by centrifugation, suspended with 2 ml of PBS (with measured OD₂₈₀=7); glycerine was added to the final concentration of 20%; and the resultant was subpackaged at 0.5 ml/tube and cryopreserved at -80°C.

### Inoculation in deep-well plate and ITPG induction of Fab expression:

The colonies (one piece of deep-well plate) from the large dish on which 0.4 ml of the bacterial solution was spread in the first round of screening of the heavy-chain replacement phage library were inoculated; and the processes of the preparation of reagents and materials as well as the Fab induction and expression were the same as those for the light-chain replacement clones.

ELISA analysis and screening of CD40 antigen-specific heavy-chain replacement positive clones:
the screening process was the same as that for the light-chain replacement clones.

The clones with ELISA readings greater than 0.8 were sequenced and analyzed to obtain 28 heavy-chain replacement positive clones, which were cryopreserved at -80°C.

### Example 8: Screening of Light- and Heavy-chain Double-replacement Positive Clones of CD40 Antigen-specific Selicrelumab

Light-chain fragments of the light-chain replacement positive clones (see FIG. 2, third polynucleotide) and heavy-chain variable region fragments of the heavy-chain replacement positive clones (see FIG. 2, fourth polynucleotide) were prepared as follows:
The cryopreserved bacterial solutions of 37 light-chain replacement positive clones (Example 6) and 28 heavy-chain replacement positive clones (Example 7) were thawed at room temperature; 5 µl of each bacterial solution was taken and mixed to obtain a CD40-LC-ZH-M37 bacterial solution and a CD40-VH-ZH-M28 bacterial solution. 50 µl of each bacterial solution was mixed with 500 µl of 2YT-Amp, then spread on a large dish, and then cultured overnight at 32°C.

Next day, all the colonies were collected from both large dishes. The colonies were cryopreserved at -80°C in two tubes for each bacterial solution.

The vector DNAs were slightly extracted from CD40-VH-ZH-M28 (26 µg) and CD40-LC-ZH-M37(32 µg).

5 µg of CD40-VH-ZH-M28 vector DNAs were digested with Esp3I and SfiI; M28-VH fragments (324ng) were analyzed by electrophoresis and purified (see FIG. 2, fourth polynucleotide).

5 µg of CD40-LC-ZH-M37 vector DNAs were digested with SfiI, and M37-LC fragments (432 ng) were analyzed by electrophoresis and purified (see FIG. 2, third polynucleotide).

### Construction of light- and heavy-chain double replacement library:

The prepared M37-LC insertion fragments (third polynucleotide) and M28-VH insertion fragments (fourth polynucleotide) were subjected to four-fragment linkage with the third and fourth polynucleotides indicated in FIG. 2 to construct light- and heavy-chain double-replacement genes. The four fragments in a total amount of 596 ng were mixed at equal molecular number ratio of 1:1:1:1 and linked at 20°C for 4 h.

A linkage product was purified to obtain 450 ng of light- and heavy-chain double-replacement gene library.

50 ng of the purified linkage product was used to transform one tube of TG1 electrocompetent cells, which was then titrated, spread on a dish, and cultured overnight at 32°C.

Next day, the capacity of the light- and heavy-chain double-replacement bacteria library was analyzed and calculated as 2×10⁶.

Colony inoculation and Fab expression induction:
two pieces of deep-well plates were inoculated with the colonies, and then induced and cultured overnight with IPTG at 30°C. Two ELISA plates were coated with CD40 antigens at 100 ng/well overnight at 4°C.

ELISA analysis and screening of CD40 antigen-specific light- and heavy-chain double-replacement positive clones:
the analysis and screening process was the same as that for the single-replacement clones.

The positive clones with ELISA readings greater than 1.5 were sequenced and analyzed to obtain 10 unique new VHs and 19 unique new LCs, and VH and LC were combined to obtain 30 double-replacement positive clones, which were cryopreserved at -80°C.

### Example 9: Functional Analysis of Light-chain Replacement, Heavy-chain Replacement, and Light- and Heavy-chain Double-replacement Positive Clones of CD40 Antigen-specific Selicrelumab

### Antibody expression:

It was selected and determined to express and purify, by 293 cell transient transfection, the full-length antibodies (KLC/IgG1) of 16 (#2-17) screened CD40 antigen-specific positive clones with ELISA readings equivalent to those of the positive control, and meanwhile, the reference antibody Selicrelumab (#1) was expressed as a positive control.
1 positive control: Selicrelumab, #1;
6 light-chain replacement clones: #2-7;
6 heavy-chain replacement clones: #8-13;
4 light- and heavy-chain double replacement clones: #14-17;
15 purified antibodies were obtained in total (Clones #8 and #17 were not expressed).

The details were shown in Table 1.

**Table 1**

| **Antibody expression no.** | **Antibody expression type** | **ELISA reading** |
|---|---|---|
| Positive antibody | Selicrelumab | 2.923 |
| DDBK003-2 | Light-chain replacement | 3.148 |
| DDBK003-3 | Light-chain replacement | 2.872 |
| DDBK003-4 | Light-chain replacement | 2.903 |
| DDBK003-5 | Light-chain replacement | 3.11 |
| DDBK003-6 | Light-chain replacement | 3.224 |
| DDBK003-7 | Light-chain replacement | 3.099 |
| DDBK003-9 | Heavy-chain replacement | 3.205 |
| DDBK003-10 | Heavy-chain replacement | 3.13 |
| DDBK003-11 | Heavy-chain replacement | 3.175 |
| DDBK003-12 | Heavy-chain replacement | 3.222 |
| DDBK003-13 | Heavy-chain replacement | 3.326 |
| DDBK003-14 | Light- and heavy-chain double replacement | 2.025 |
| DDBK003-15 | Light- and heavy-chain double replacement | 1.839 |
| DDBK003-16 | Light- and heavy-chain double replacement | 1.93 |

### Antibody excitation activity analysis:

Referring to the reference (Scandinavian Journal of Immunology 65, 479-486), the antibody and human PBMC were co-cultured for five days (antibody concentration: 100 nM); the IFN-γ concentration (pg/ml) of the supernatant of the culture solution was detected; and the excitation activity of 15 antibodies were analyzed. The results showed that, compared with the positive antibody Selicrelumab (#1), all the 14 screened antibodies showed the excitation activity to different degrees, and the excitation activity of #2, 3, 4, 5, 6, 7 and #16 were equivalent to that of the positive control antibody.

The results were shown in FIG. 4.

Vhs and full-length light-chain amino acid sequences of six chain replacement functional antibodies:
The amino acid sequence alignment of two light-chain replacement clones #2 and #3, two heavy-chain replacement clones #12 and #13, and two light- and heavy-chain double-replacement clones #14 and #16 showed that, compared with the corresponding light-chain or heavy-chain amino acid sequence of the positive control antibody Selicrelumab, the amino acid sequences of the CDRs of the screened light chains (light-chain replacement clones), heavy chains (heavy-chain replacement clones), and light and heavy chains (light- and heavy-chain double-replacement clones) showed a significant difference. See Table 2.

**Table 2**

| Clone No. | SEQ ID NO. | | | | | | | |
|---|---|---|---|---|---|---|---|---|
| | HCDR1 | HCDR2 | HCDR3 | LCDR1 | LCDR2 | LCDR3 | VH | Light chain |
| DDBK003-2 | 16 | 17 | 18 | 20 | 21 | 22 | 15 | 19 |
| DDBK003-3 | 16 | 17 | 18 | 24 | 25 | 26 | 15 | 23 |
| DDBK003-12 | 28 | 29 | 30 | 31 | 32 | 33 | 27 | 11 |
| DDBK003-13 | 35 | 36 | 37 | 31 | 32 | 33 | 34 | 11 |
| DDBK003-14 | 39 | 40 | 41 | 43 | 32 | 44 | 38 | 42 |
| DDBK003-16 | 39 | 40 | 41 | 46 | 47 | 48 | 38 | 45 |

### Example10: Screening of Light-chain Replacement Positive Clones of Her3 Antigen-specific Patritumab

Referring to the screening process of Example 6, the light-chain replacement positive clones and heavy-chain replacement positive clones were screened for Her3 antigen-specific Patritumab.

One tube of light-chain replacement phage library (Example 4) was screened for the first round, and the output of the light-chain replacement phages was 1 × 10⁵.

The bacteria were collected, amplified and packaged for the first round of output to obtain the first round of output of light-chain replacement phage library, and second and third rounds of screening were conducted. The output of the light-chain replacement phages from the third round of screening was 2.2×10⁷.

The light-chain replacement phage library clones were inoculated in two pieces of deep-well plates, and cultured overnight at 32°C and 250 rpm; and Fab expression was induced by IPTG

ELISA analysis was conducted to screen Her3 antigen-specific light-chain replacement positive clones; and 51 clones with high OD₄₀₅ were sequenced to obtain 28 unique light-chain replacement positive clones.

### Example11: Functional Analysis of Light-chain Replacement Positive Clones of Her3 Antigen-specific Patritumab

### Antibody expression:

It was selected and determined to express and purify, by 293 cell transient transfection, the full-length antibodies (#2-13, KLC/IgG1) of Patritumab (#1) and 12 screened Her3 antigen-specific light-chain replacement positive clones with ELISA readings equivalent to those of the positive control.

### Antibody endocytosis activity analysis:

A gastric cancer organoid (3-27-T) was recovered and cultured for 7 days: the cultured gastric cancer organoid was collected, washed to remove matrigel, suspended to a proper volume with a phenol red-free D-Hanks suspension, and suspended and mixed uniformly; and the organoid was subpackaged into a 96-well plate at 50 µL per well.

Referring to the reference (ThermoFisher/Invitrogen, Zenon^{™} pHrodo^{™} iFL IgG Labeling Reagents, Catalog Nos. Z25609, Z25610, Z25611, Z25612, Pub. No. MAN0017436 Rev. B.0), 160 nM antibody solution and 4x coupling reagent were formulated, mixed at a ratio of 1:1, and incubated for 5 min at room temperature.

The antibody-coupling agent mixed solution was added at 50 µl per well to the 96-well plate in which the gastric cancer organoid was subpackaged, and incubated for 60 min at room temperature.

Microscopic examination was conducted, the form of the fluorescent field was recorded by photography, and the endocytosis activities of the antibodies were analyzed.

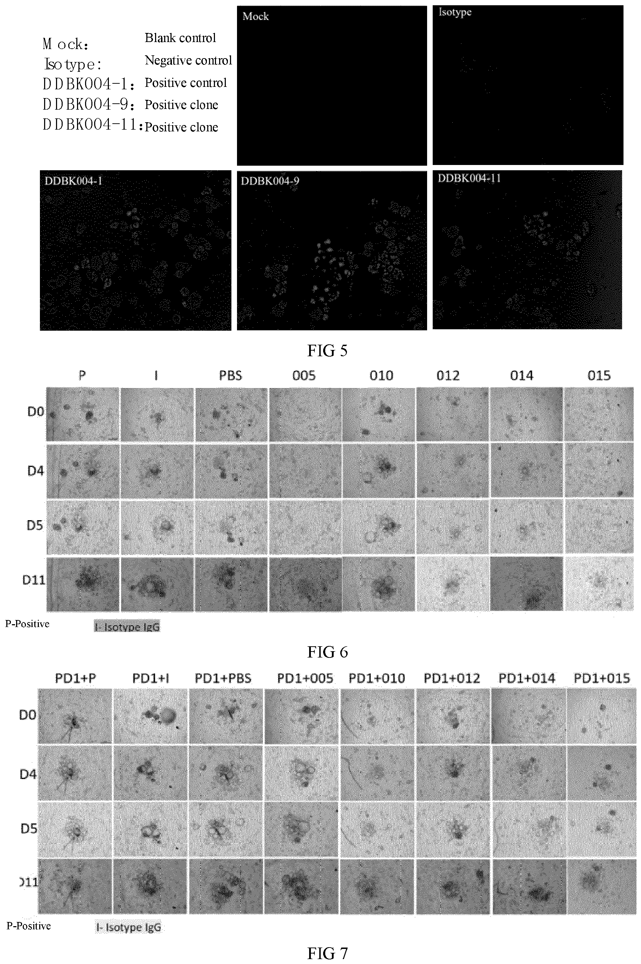

The results were shown in FIG. 5. The results showed that four antibodies including DDBK004-2, DDBK004-3, DDBK004-7 and DDBK004-13 showed green fluorescence, indicating the presence of endocytosis activity, which is, however, weaker than that of the positive control; DDBK004-11 showed the fluorescence intensity equivalent to that of the positive control, indicating that this antibody had the endocytosis activity equivalent to that of the positive antibody; and DDBK004-9 showed the highest fluorescence intensity higher than that of the positive control, indicating that this antibody had the endocytosis activity higher than that of the positive control.

The CDR and variable region sequences of the above antibodies were shown in Table 3:

**Table 3**

| Clone No. | SEQ ID NO. | | | | |
|---|---|---|---|---|---|
| | LCDR1 | LCDR2 | LCDR3 | VH | Light chain |
| DDBK004-9 | 50 | 51 | 52 | 53 | 49 |
| DDBK004-11 | 55 | 51 | 56 | 53 | 54 |

### Example 12: Screening of Light-chain Replacement Positive Clones, Heavy-chain Replacement Positive Clones and Double-replacement Positive Clones of TIGIT Antigen-specific Reference Antibody Tiragolumab, and Analysis of Biological Function of Positive Clones

### Screening of chain replacement positive clones:

Referring to preparation of antigens in Example 6, TIGIT antigen-specific light-chain replacement positive clones and heavy-chain replacement positive clones were screened from the light-chain replacement phage library and the heavy-chain replacement phage library.

Nearly 1000 clones were analyzed by ELISA. Based on ELISA analysis results, and referring to the OD₄₀₅ readings of the positive control clones, more than 400 clones with higher readings were sequenced to determine the TIGIT antigen-specific light-chain replacement positive clones and heavy-chain replacement positive clones.

Referring to Example 8, the light-chain replacement positive clones with unique sequences and the heavy-chain replacement positive clones with unique sequences were selected to prepare unique-sequence light-chain fragments (see FIG. 2, third polynucleotide) and heavy-chain fragments (see FIG. 2, fourth polynucleotide); both fragments were subjected four-fragment linkage with the third and fourth vector polynucleotides indicated in FIG. 2, to construct a light- and heavy-chain double-replacement gene library, bacteria library and phage library, from which double-replacement positive clones with unique sequences were screened.

It was selected and determined to express and purify, by 293 cell transient transfection, the full-length antibodies (KLC/IgG1) of 15 (#2-16) screened TIGIT antigen-specific positive clones with ELISA readings equivalent to or higher than those of the positive control, and meanwhile, the reference antibody Tiragolumab (#1) was expressed as a positive control. 15 purified antibodies were obtained in total (Clone #3 was not expressed), as shown in Table 4:

**Table 4**

| **Antibody Expression No.** | **Screened Clone No.** | **Antibody Type** | **ELISA OD₄₀₅ Reading** |
|---|---|---|---|
| 1 | DDBJY38/39 | Positive control | 0.499-2.867 |
| 2 | TT128 | Light-chain replacement | 2.949 |
| 3 | TT261 | Light-chain replacement | 3.349 |
| 4 | TT194 | Light-chain replacement | 2.89 |
| 5 | TT233 | Light-chain replacement | 3.139 |
| 6 | TT216 | Light-chain replacement | 3.122 |
| 7 | TT210 | Light-chain replacement | 2.84 |
| 8 | TT174 | Light-chain replacement | 3.249 |
| 9 | TT255 | Light-chain replacement | 3.173 |
| 10 | TT321 | Heavy-chain replacement | 0.547 |
| 11 | TT335 | Heavy-chain replacement | 0.669 |
| 12 | TT357 | Heavy-chain replacement | 0.471 |
| 13 | TT364 | Heavy-chain replacement | 1.165 |
| 14 | TT385 | Heavy-chain replacement | 0.474 |
| 15 | TT397 | Light- and heavy-chain double replacement | 0.91 |
| 16 | TT400 | Light- and heavy-chain double replacement | 0.452 |

The amino acid sequence of Tiragolumab could be found in WO2009126688A2.

### Analysis of tumor cell killing activity of antibody:

The killing activity of the combination of a TIGIT antibody and a PD-1 antibody (Pembrolizomab) to non-small cell lung cancer organoids was observed through experiments. Based on the results of multiple pre-experiments, five antibodies numbered #5, 10, 12, 14, and 15 in Table 4 showed the killing activity to different degrees. In order to compare and further verify the results of the pre-experiments, a TIGIT/PD-1 antibody combination group (1-8) and a single TIGIT antibody group (9-16) were set up for the experiment, with 8 wells in each group.

### Experimental procedures were as follows:

1, Referring to the reference (Tumor organoid-T-cell coculture systems; NATURE PROTOCOLS! VOL 15 JANUARY 2020 15-39), a non-small cell lung cancer organoid (4-11-T) was recovered, and cultured for 24 h in a culture solution containing IFNγ (200 ng/mL), collected, washed to remove matrigel, suspended with the culture solution to a proper volume, and then uniformly mixed for later use.
2, PBMC (4-11-PBMC) was counted to calculate the vitality and cell counts, collected and washed, suspended with a T-cell culture solution (RPMI1640, 10%FBS, 1% double-antibody, 2 mM glutamine, adding IL2) and adjusted to a proper concentration, and dispensed to a 96-well U-shaped plate at 5×10³PBMC/well.
3, A proper amount of non-small cell lung cancer organoid was added to each well, and the final volume was adjusted to 200 µL/well.
4, The PD-1 antibody (Pembrolizomab) was added to the wells 1 to 8, with the final concentration of 100 nM; then, from well 1 to well 8, a positive control antibody (P), an Isotype antibody (I), PBS and five pre-experiment positive clone antibodies were added in sequence from the well 1 to the well 8, and PBS was added to one well as a blank control. For the wells 9 to 16, the PD-1 antibody (Pembrolizomab) was not added, and other reagents were added as that for the wells 1 to 8. The results are shown in Table 5.

**Table 5**

| | | | | | | | | |
|---|---|---|---|---|---|---|---|---|
| 1-8 | PD1+P | PD1+I | PD1+PBS | PD1+005 | PD1+010 | PD1+012 | PD1+014 | PD1+015 |
| | Positive antibody | Isotype antibody | | | | | | |
| 9-16 | +P | +I | +PBS | +005 | +010 | +012 | +014 | 015 |
| | Positive antibody | Isotype antibody | | | | | | |

5, Microscopic examination was conducted continuously, and photos were taken on a regular basis to record the changes of organoid and lymphocytes; and the killing activity of the antibodies against the non-small cell lung organoid was analyzed.
6, The results of the single use of TIGIT antibody showed (FIG. 6) that, compared with the PBS blank control and the Isotype antibody, the single TIGIT antibody did not exhibit significant killing activity against the non-small cell lung cancer organoid.
7, The results of the TIGIT-PD-1 antibody combination group showed (FIG. 7) that:
1) "negative" control wells (PD1+I; PD1+PBS) did not show significant killing activity of the single PD-1 antibody against the non-small cell lung organoid;
2) positive control wells (PD1+P) showed significant killing activity of the TIGIT and PD-1 antibody combination against the non-small cell lung cancer organoid on Day 11;
3) five experimental wells, in which the TIGIT antibodies #5, #10, #12, #14 and #15 were combined with the PD-1 antibody (PD1+005, PD1+010, PD1+012, PD1+014, and PD1+015), showed an equivalent killing activity to the positive controls (PD1+P) against the non-small cell lung cancer organoid, and on Day 11 (D11), a significant killing activity was observed.

The CDR and variable region sequences of the above antibodies were shown in Table 6:

**Table 6**

| No. | Clone No. | SEQ ID NO. | | | | | | | |
|---|---|---|---|---|---|---|---|---|---|
| | | HCDR1 | HCDR2 | HCDR3 | LCDR1 | LCDR2 | LCDR3 | VH | Light chain |
| #5 | TT233 | | | | 58 | 59 | 60 | the same as Tiragolumab | 57 |
| #10 | TT321 | 62 | 63 | 64 | | | | 61 | the same as Tiragolumab |
| #12 | TT357 | 66 | 67 | 68 | | | | 65 | the same as Tiragolumab |
| #14 | TT385 | 77 | 78 | 79 | | | | 76 | the same as Tiragolumab |
| #15 | TT397 | 73 | 74 | 75 | 70 | 51 | 71 | 72 | 69 |

The foregoing detailed description is provided by way of explanation and exemplification, and is not intended to limit the scope of the appended claims. Various changes of the embodiments listed in the present application until now would be obvious to those of ordinary skills in the art, and should be kept within the scope of the appended claims and equivalents thereof.

## Claims

1. A method for selecting a functional antigen-binding protein, comprising:
a) providing a first polynucleotide comprising R1-nucleic acid fragment I-R2 in a 5'-to-3' direction, the nucleic acid fragment I being capable of encoding an antigen-binding fragment I;
b) providing a second polynucleotide comprising R3-reference nucleic acid fragment II-R4 in a 5'-to-3' direction, the reference nucleic acid fragment II being capable of encoding a reference antigen-binding fragment II, which, together with a reference antigen-binding fragment I encoded by a reference nucleic acid fragment I, is capable of forming a reference antigen-binding protein;
c) providing a first vector polynucleotide comprising R2-vector fragment I-R3 in a 5'-to-3' direction;
d) providing a second vector polynucleotide comprising R4-vector fragment II-R1 in a 5'-to-3' direction;
e) cleaving, with a restriction endonuclease, the first polynucleotide, the second polynucleotide, the first vector polynucleotide and the second vector polynucleotide to obtain the cleaved first polynucleotide, the cleaved second polynucleotide, the cleaved first vector polynucleotide and the cleaved second vector polynucleotide;
f) mixing the cleaved first polynucleotide, the cleaved second polynucleotide, the cleaved first vector polynucleotide and the cleaved second vector polynucleotide, such that the cleaved first polynucleotide, the cleaved second polynucleotide, the cleaved first vector polynucleotide and the cleaved second vector polynucleotide can be cyclized by directional linkage to form first to-be-screened vectors;
g) expressing the first to-be-screened vectors, and selecting a first to-be-screened vector capable of expressing an expression product having the following property, as a first replacement vector: capability of binding to a target, to which the reference antigen-binding protein can bind, at a capacity that is 30% or more of a binding capacity of the reference antigen-binding protein to the target; and
h) deriving the functional antigen-binding protein from the first replacement vector,
wherein R1, R2, R3 and R4 are each independently a restriction endonuclease recognition site.

2. The method according to claim 1, comprising cleaving the first polynucleotide with a restriction endonuclease specifically recognizing R1 and R2, to obtain the cleaved first polynucleotide.

3. The method according to any one of claims 1 to 2, comprising cleaving the second polynucleotide with a restriction endonuclease specifically recognizing R3 and R4, to obtain the cleaved second polynucleotide.

4. The method according to any one of claims 1 to 3, comprising cleaving the first vector polynucleotide with a restriction endonuclease specifically recognizing R2 and R3, to obtain the cleaved first vector polynucleotide.

5. The method according to any one of claims 1 to 4, comprising cleaving the second vector polynucleotide with a restriction endonuclease specifically recognizing R4 and R1, to obtain the cleaved second vector polynucleotide.

6. The method according to any one of claims 1 to 5, wherein a terminus of R1 resulting from specific cleavage by the restriction endonuclease specifically recognizing R1 and a terminus of any one of R2, R3 and R4 resulting from specific cleavage by the corresponding restriction endonuclease do not recognize or link to each other.

7. The method according to any one of claims 1 to 6, wherein a terminus of R2 resulting from specific cleavage by the restriction endonuclease specifically recognizing R2 and a terminus of any one of R1, R3 and R4 resulting from specific cleavage by the corresponding restriction endonuclease do not recognize or link to each other.

8. The method according to any one of claims 1 to 7, wherein a terminus of R3 resulting from specific cleavage by the restriction endonuclease specifically recognizing R3 and a terminus of any one of R1, R2 and R4 resulting from specific cleavage by the corresponding restriction endonuclease do not recognize or link to each other.

9. The method according to any one of claims 1 to 8, wherein a terminus of R4 resulting from specific cleavage by the restriction endonuclease specifically recognizing R4 and a terminus of any one of R1, R2 and R3 resulting from specific cleavage by the corresponding restriction endonuclease do not recognize or link to each other.

10. The method according to any one of claims 1 to 9, wherein the restriction endonuclease is selected from the group consisting of SfiI and BsmBI.

11. The method according to any one of claims 1 to 10, comprising introducing the first to-be-screened vector into a cell to express the first to-be-screened vector.

12. The method according to any one of claims 1 to 11, comprising introducing the first to-be-screened vector into a bacterium to prepare a phage library comprising one or more of the first to-be-screened vectors, thereby obtaining the functional antigen-binding protein from the phage library.

13. The method according to any one of claims 1 to 12, wherein the vector fragment I comprises a linker, and the vector fragment II is derived from a display vector.

14. The method according to claim 13, wherein the nucleic acid fragment I encodes an antibody light chain or a fragment thereof; the vector fragment I comprises the linker; the reference nucleic acid fragment II encodes an antibody heavy chain or a fragment thereof; and the vector fragment II is derived from the display vector.

15. The method according to any one of claims 13 to 14, wherein R1 comprises a nucleotide sequence as set forth in SEQ ID NO: 1.

16. The method according to any one of claims 13 to 15, wherein R2 comprises a nucleotide sequence as set forth in SEQ ID NO: 2.

17. The method according to any one of claims 13 to 16, wherein R3 comprises a nucleotide sequence as set forth in SEQ ID NO: 3.

18. The method according to any one of claims 13 to 17, wherein R4 comprises a nucleotide sequence as set forth in SEQ ID NO: 4.

19. The method according to any one of claims 1 to 12, wherein the vector fragment II comprises a linker, and the vector fragment I is derived from a display vector.

20. The method according to claim 19, wherein the nucleic acid fragment I encodes an antibody heavy chain or a fragment thereof; the vector fragment I is derived from the display vector; the reference nucleic acid fragment II encodes an antibody light chain or a fragment thereof; and the vector fragment II comprises the linker.

21. The method according to any one of claims 19 to 20, wherein R1 comprises a nucleotide sequence as set forth in SEQ ID NO: 3.

22. The method according to any one of claims 19 to 21, wherein R2 comprises a nucleotide sequence as set forth in SEQ ID NO: 4.

23. The method according to any one of claims 19 to 22, wherein R3 comprises a nucleotide sequence as set forth in SEQ ID NO: 1.

24. The method according to any one of claims 19 to 23, wherein R4 comprises a nucleotide sequence as set forth in SEQ ID NO: 2.

25. The method according to any one of claims 13 to 24, wherein the display vector is derived from a pComb3x vector.

26. The method according to any one of claims 13 to 25, wherein the linker comprises a nucleic acid sequence encoding a signal peptide pelB or a fragment thereof.

27. The method according to any one of claims 13 to 26, wherein the linker has a length of about 50 bases to about 200 bases.

28. The method according to any one of claims 1 to 11, comprising introducing the first to-be-screened vector into a bacterium to obtain a DNA of the first to-be-screened vector from the bacterium, and introducing the DNA of the first to-be-screened vector into a cell, from which a functional antigen-specific binding polypeptide is obtained.

29. The method according to claim 28, wherein the cell is a mammalian cell.

30. The method according to any one of claims 28 to 29, wherein the vector fragment I and/or the vector fragment II are/is derived from a mammalian cell expression vector.

31. The method according to any one of claims 28 to 30, wherein the mammalian cell expression vector is derived from pDGB4.

32. The method according to any one of claims 28 to 31, wherein the nucleic acid fragment I encodes an antibody light chain or a fragment thereof; and the reference nucleic acid fragment II encodes an antibody heavy chain or a fragment thereof.

33. The method according to any one of claims 28 to 32, wherein R1 comprises a nucleotide sequence as set forth in SEQ ID NO: 7.

34. The method according to any one of claims 28 to 33, wherein R2 comprises a nucleotide sequence as set forth in SEQ ID NO: 8.

35. The method according to any one of claims 28 to 34, wherein R3 comprises a nucleotide sequence as set forth in SEQ ID NO: 5.

36. The method according to any one of claims 28 to 35, wherein R4 comprises a nucleotide sequence as set forth in SEQ ID NO: 6.

37. The method according to any one of claims 28 to 31, wherein the nucleic acid fragment I encodes an antibody heavy chain or a fragment thereof; and the reference nucleic acid fragment II encodes an antibody light chain or a fragment thereof.

38. The method according to claim 37, wherein R1 comprises a nucleotide sequence as set forth in SEQ ID NO: 5.

39. The method according to any one of claims 37 to 38, wherein R2 comprises a nucleotide sequence as set forth in SEQ ID NO: 6.

40. The method according to any one of claims 37 to 39, wherein R3 comprises a nucleotide sequence as set forth in SEQ ID NO: 7.

41. The method according to any one of claims 37 to 40, wherein R4 comprises a nucleotide sequence as set forth in SEQ ID NO: 8.

42. The method according to any one of claims 1 to 41, comprising:
a) providing a third polynucleotide comprising R5-nucleic acid fragment I'-R6 in a 5'-to-3' direction;
b) providing a fourth polynucleotide comprising R7-nucleic acid fragment II'-R8 in a 5'-to-3' direction, wherein the nucleic acid fragment II' is capable of encoding an antigen-binding protein II', and an antigen-binding fragment II', together with the reference antigen-binding fragment I, is capable of forming an antigen-binding protein having the following property: capability of binding to a target, to which the reference antigen-binding protein can bind, at a capacity that is 30% or more of a binding capacity of the reference antigen-binding protein to the target;
c) providing a third vector polynucleotide comprising R6-vector fragment III-R7 in a 5'-to-3' direction;
d) providing a fourth vector polynucleotide comprising R8-vector fragment IV-R5 in a 5'-to-3' direction;
e) cleaving, with a restriction endonuclease, the third polynucleotide, the fourth polynucleotide, the third vector polynucleotide and the fourth vector polynucleotide to obtain the cleaved third polynucleotide, the cleaved fourth polynucleotide, the cleaved third vector polynucleotide and the cleaved fourth vector polynucleotide;
f) mixing the cleaved third polynucleotide, the cleaved fourth polynucleotide, the cleaved third vector polynucleotide and the cleaved fourth vector polynucleotide, such that the cleaved third polynucleotide, the cleaved fourth polynucleotide, the cleaved third vector polynucleotide and the cleaved fourth vector polynucleotide can be cyclized by directional linkage to form double-replacement to-be-screened vectors;
g) expressing the double-replacement to-be-screened vectors, and selecting a double-replacement to-be-screened vector capable of expressing an expression product having the following property, as a double-replacement vector: capability of binding to a target at a capacity that is 30% or more of a binding capacity of the reference antigen-binding protein to the target; and
h) deriving the functional antigen-binding protein from the double-replacement vector,
wherein R5, R6, R7 and R8 are each independently the restriction endonuclease recognition site.

43. The method according to claim 42, comprising cleaving the third polynucleotide with a restriction endonuclease specifically recognizing R5 and R6, to obtain the cleaved third polynucleotide.

44. The method according to any one of claims 42 to 43, comprising cleaving the fourth polynucleotide with a restriction endonuclease specifically recognizing R7 and R8, to obtain the cleaved fourth polynucleotide.

45. The method according to any one of claims 42 to 44, comprising cleaving the third vector polynucleotide with a restriction endonuclease specifically recognizing R6 and R7, to obtain the cleaved third vector polynucleotide.

46. The method according to any one of claims 42 to 45, comprising cleaving the fourth vector polynucleotide with a restriction endonuclease specifically recognizing R8 and R5, to obtain the cleaved fourth vector polynucleotide.

47. The method according to any one of claims 42 to 46, wherein a terminus of R5 resulting from specific cleavage by the restriction endonuclease specifically recognizing R5 and a terminus of any one of R6, R7 and R8 resulting from specific cleavage by the corresponding restriction endonuclease do not recognize or link to each other.

48. The method according to any one of claims 42 to 47, wherein a terminus of R6 resulting from specific cleavage by the restriction endonuclease specifically recognizing R6 and a terminus of any one of R5, R7 and R8 resulting from specific cleavage by the corresponding restriction endonuclease do not recognize or link to each other.

49. The method according to any one of claims 42 to 48, wherein a terminus of R7 resulting from specific cleavage by the restriction endonuclease specifically recognizing R7 and a terminus of any one of R5, R6 and R8 resulting from specific cleavage by the corresponding restriction endonuclease do not recognize or link to each other.

50. The method according to any one of claims 42 to 49, wherein a terminus of R8 resulting from specific cleavage by the restriction endonuclease specifically recognizing R8 and a terminus of any one of R5, R6 and R7 resulting from specific cleavage by the corresponding restriction endonuclease do not recognize or link to each other.

51. The method according to any one of claims 42 to 50, wherein the restriction endonuclease is selected from the group consisting of SfiI and BsmBI.

52. The method according to any one of claims 42 to 51, comprising introducing the double-replacement to-be-screened vector into a cell to express the double-replacement to-be-screened vector.

53. The method according to any one of claims 42 to 52, comprising introducing the double-replacement to-be-screened vector into a bacterium to prepare a phage library comprising one or more of the double-replacement to-be-screened vectors, thereby obtaining the functional antigen-binding protein from the phage library.

54. The method according to any one of claims 42 to 53, wherein the vector fragment III comprises a linker, and the vector fragment IV is derived from a display vector.

55. The method according to claim 54, wherein the nucleic acid fragment I' encodes an antibody light chain or a fragment thereof; the vector fragment III comprises the linker; the nucleic acid fragment II' encodes an antibody heavy chain or a fragment thereof; and the vector fragment IV is derived from the display vector.

56. The method according to any one of claims 42 to 55, wherein R5 comprises a nucleotide sequence as set forth in SEQ ID NO: 1.

57. The method according to any one of claims 42 to 56, wherein R6 comprises a nucleotide sequence as set forth in SEQ ID NO: 2.

58. The method according to any one of claims 42 to 57, wherein R7 comprises a nucleotide sequence as set forth in SEQ ID NO: 3.

59. The method according to any one of claims 42 to 58, wherein R8 comprises a nucleotide sequence as set forth in SEQ ID NO: 4.

60. The method according to any one of claims 42 to 53, wherein the vector fragment IV comprises a linker, and the vector fragment III is derived from a display vector.

61. The method according to claim 60, wherein the nucleic acid fragment I' encodes an antibody heavy chain or a fragment thereof; the vector fragment III is derived from the display vector; the nucleic acid fragment II' encodes an antibody light chain or a fragment thereof; and the vector fragment IV comprises the linker.

62. The method according to any one of claims 60 to 61, wherein R5 comprises a nucleotide sequence as set forth in SEQ ID NO: 3.

63. The method according to any one of claims 60 to 62, wherein R6 comprises a nucleotide sequence as set forth in SEQ ID NO: 4.

64. The method according to any one of claims 60 to 63, wherein R7 comprises a nucleotide sequence as set forth in SEQ ID NO: 1.

65. The method according to any one of claims 60 to 64, wherein R8 comprises a nucleotide sequence as set forth in SEQ ID NO: 2.

66. The method according to any one of claims 54 to 65, wherein the display vector is derived from a pComb3x vector.

67. The method according to any one of claims 54 to 66, wherein the linker comprises a nucleic acid sequence encoding a signal peptide pelB or a fragment thereof.

68. The method according to any one of claims 54 to 67, wherein the linker has a length of about 50 bases to about 200 bases.

69. The method according to any one of claims 42 to 52, comprising introducing the double-display to-be-screened vector into a bacterium to obtain a DNA of the double-display to-be-screened vector from the bacterium, and introducing the DNA of the double-display to-be-screened vector into a cell, from which functional antigen-specific binding polypeptide is obtained.

70. The method according to claim 69, wherein the cell is a mammalian cell.

71. The method according to any one of claims 69 to 70, wherein the vector fragment III and/or the vector fragment IV are/is derived from a mammalian expression vector.

72. The method according to claim 71, wherein the mammalian expression vector is derived from pDGB4.

73. The method according to any one of claims 69 to 72, wherein the nucleic acid fragment I' encodes an antibody light chain or a fragment thereof; and the nucleic acid fragment II' encodes an antibody heavy chain or a fragment thereof.

74. The method according to any one of claims 69 to 73, wherein R5 comprises a nucleotide sequence as set forth in SEQ ID NO: 7.

75. The method according to any one of claims 69 to 74, wherein R6 comprises a nucleotide sequence as set forth in SEQ ID NO: 8.

76. The method according to any one of claims 69 to 75, wherein R7 comprises a nucleotide sequence as set forth in SEQ ID NO: 5.

77. The method according to any one of claims 69 to 76, wherein R8 comprises a nucleotide sequence as set forth in SEQ ID NO: 6.

78. The method according to any one of claims 69 to 72, wherein the nucleic acid fragment I' encodes an antibody heavy chain or a fragment thereof; and the nucleic acid fragment II' encodes an antibody light chain or a fragment thereof.

79. The method according to claim 78, wherein R5 comprises a nucleotide sequence as set forth in SEQ ID NO: 5.

80. The method according to any one of claims 78 to 79, wherein R6 comprises a nucleotide sequence as set forth in SEQ ID NO: 6.

81. The method according to any one of claims 78 to 80, wherein R7 comprises a nucleotide sequence as set forth in SEQ ID NO: 7.

82. The method according to any one of claims 78 to 81, wherein R8 comprises a nucleotide sequence as set forth in SEQ ID NO: 8.

83. The method according to any one of claims 1 to 82, comprising:
a) providing a fifth polynucleotide comprising R9-reference nucleic acid fragment I-R10 in a 5'-to-3' direction;
b) providing a sixth polynucleotide comprising R11-nucleic acid fragment II-R12 in a 5'-to-3' direction;
c) providing a fifth vector polynucleotide comprising R10-vector fragment V-R11 in a 5'-to-3' direction;
d) providing a sixth vector polynucleotide comprising R12-vector fragment VI-R9 in a 5'-to-3' direction;
e) cleaving, with a restriction endonuclease, the fifth polynucleotide, the sixth polynucleotide, the fifth vector polynucleotide and the sixth vector polynucleotide to obtain the cleaved fifth polynucleotide, the cleaved sixth polynucleotide, the cleaved fifth vector polynucleotide and the cleaved sixth vector polynucleotide;
f) mixing the cleaved fifth polynucleotide, the cleaved sixth polynucleotide, the cleaved fifth vector polynucleotide and the cleaved sixth vector polynucleotide, such that the cleaved fifth polynucleotide, the cleaved sixth polynucleotide, the cleaved fifth vector polynucleotide and the cleaved sixth vector polynucleotide can be cyclized by directional linkage to form second to-be-screened vectors;
g) expressing the second to-be-screened vectors, and selecting a second to-be-screened vector capable of expressing an expression product having the following property, as a second replacement vector: capability of binding to a target, to which the reference antigen-binding protein can bind, at a capacity that is 30% or more of a binding capacity of the reference antigen-binding protein to the target; and
h) selecting a nucleic acid fragment II of the second replacement vector as the nucleic acid fragment II',
wherein R9, R10, R11 and R12 are each independently a restriction endonuclease recognition site.

84. The method according to claim 83, comprising cleaving the fifth polynucleotide with a restriction endonuclease specifically recognizing R9 and R10, to obtain the cleaved fifth polynucleotide.

85. The method according to any one of claims 83 to 84, comprising cleaving the sixth polynucleotide with a restriction endonuclease specifically recognizing R11 and R12, to obtain the cleaved sixth polynucleotide.

86. The method according to any one of claims 83 to 85, comprising cleaving the fifth vector polynucleotide with a restriction endonuclease specifically recognizing R10 and R11, to obtain the cleaved fifth vector polynucleotide.

87. The method according to any one of claims 83 to 86, comprising cleaving the sixth vector polynucleotide with a restriction endonuclease specifically recognizing R12 and R9, to obtain the cleaved sixth vector polynucleotide.

88. The method according to any one of claims 83 to 87, wherein a terminus of R9 resulting from specific cleavage by the restriction endonuclease specifically recognizing R9 and a terminus of any one of R10, R11 and R12 resulting from specific cleavage by the corresponding restriction endonuclease do not recognize or link to each other.

89. The method according to any one of claims 83 to 88, wherein a terminus of R10 resulting from specific cleavage by the restriction endonuclease specifically recognizing R10 and a terminus of any one of R11, R12 and R9 resulting from specific cleavage by the corresponding restriction endonuclease do not recognize or link to each other.

90. The method according to any one of claims 83 to 89, wherein a terminus of R11 resulting from specific cleavage by the restriction endonuclease specifically recognizing R11 and a terminus of any one of R9, R10 and R12 resulting from specific cleavage by the corresponding restriction endonuclease do not recognize or link to each other.

91. The method according to any one of claims 83 to 90, wherein a terminus of R12 resulting from specific cleavage by the restriction endonuclease specifically recognizing R12 and a terminus of any one of R9, R10 and R11 resulting from specific cleavage by the corresponding restriction endonuclease do not recognize or link to each other.

92. The method according to any one of claims 83 to 91, wherein the restriction endonuclease is selected from the group consisting of SfiI and BsmBI.

93. The method according to any one of claims 83 to 92, comprising introducing the second to-be-screened vector into a cell to express the second to-be-screened vector.

94. The method according to any one of claims 83 to 93, comprising introducing the second to-be-screened vector into a bacterium to prepare a phage library comprising one or more of the second to-be-screened vectors, thereby obtaining the functional antigen-binding protein from the phage library.

95. The method according to any one of claims 83 to 94, wherein the vector fragment V comprises a linker, and the vector fragment IV is derived from a display vector.

96. The method according to claim 95, wherein the reference nucleic acid fragment I encodes an antibody light chain or a fragment thereof; the vector fragment V comprises the linker; the nucleic acid fragment II encodes an antibody heavy chain or a fragment thereof; and the vector fragment VI is derived from the display vector.

97. The method according to any one of claims 83 to 96, wherein R9 comprises a nucleotide sequence as set forth in SEQ ID NO: 1.

98. The method according to any one of claims 83 to 97, wherein R10 comprises a nucleotide sequence as set forth in SEQ ID NO: 2.

99. The method according to any one of claims 83 to 98, wherein R11 comprises a nucleotide sequence as set forth in SEQ ID NO: 3.

100. The method according to any one of claims 83 to 99, wherein R12 comprises a nucleotide sequence as set forth in SEQ ID NO: 4.

101. The method according to any one of claims 83 to 94, wherein the vector fragment VI comprises a linker, and the vector fragment V is derived from a display vector.

102. The method according to claim 101, wherein the reference nucleic acid fragment I encodes an antibody heavy chain or a fragment thereof; the vector fragment V is derived from the display vector; the nucleic acid fragment II encodes an antibody light chain or a fragment thereof; and the vector fragment VI comprises the linker.

103. The method according to any one of claims 101 to 102, wherein R9 comprises a nucleotide sequence as set forth in SEQ ID NO: 3.

104. The method according to any one of claims 101 to 103, wherein R10 comprises a nucleotide sequence as set forth in SEQ ID NO: 4.

105. The method according to any one of claims 101 to 104, wherein R11 comprises a nucleotide sequence as set forth in SEQ ID NO: 1.

106. The method according to any one of claims 101 to 105, wherein R12 comprises a nucleotide sequence as set forth in SEQ ID NO: 2.

107. The method according to any one of claims 95 to 106, wherein the display vector is derived from a pComb3x vector.

108. The method according to any one of claims 95 to 107, wherein the linker comprises a nucleic acid sequence encoding a signal peptide pelB or a fragment thereof.

109. The method according to any one of claims 95 to 108, wherein the linker has a length of about 50 bases to about 200 bases.

110. The method according to any one of claims 83 to 93, comprising introducing the second to-be-screened vector into a bacterium to obtain a DNA of the second to-be-screened vector from the bacterium, and introducing the DNA of the second to-be-screened vector into a cell, from which a functional antigen-specific binding polypeptide is obtained.

111. The method according to claim 110, wherein the cell is a mammalian cell.

112. The method according to any one of claims 110 to 111, wherein the vector fragment V and/or the vector fragment VI are/is derived from a mammalian expression vector.

113. The method according to claim 112, wherein the mammalian expression vector is derived from pDGB4.

114. The method according to any one of claims 110 to 113, wherein the reference nucleic acid fragment I encodes an antibody light chain or a fragment thereof; and the reference nucleic acid fragment II encodes an antibody heavy chain or a fragment thereof.

115. The method according to any one of claims 110 to 114, wherein R9 comprises a nucleotide sequence as set forth in SEQ ID NO: 7.

116. The method according to any one of claims 110 to 115, wherein R10 comprises a nucleotide sequence as set forth in SEQ ID NO: 8.

117. The method according to any one of claims 110 to 116, wherein R11 comprises a nucleotide sequence as set forth in SEQ ID NO: 5.

118. The method according to any one of claims 110 to 117, wherein R12 comprises a nucleotide sequence as set forth in SEQ ID NO: 6.

119. The method according to any one of claims 110 to 113, wherein the reference nucleic acid fragment I encodes an antibody heavy chain or a fragment thereof; and the nucleic acid fragment II encodes an antibody light chain or a fragment thereof.

120. The method according to claim 119, wherein R9 comprises a nucleotide sequence as set forth in SEQ ID NO: 5.

121. The method according to any one of claims 119 to 120, wherein R10 comprises a nucleotide sequence as set forth in SEQ ID NO: 6.

122. The method according to any one of claims 119 to 121, wherein R11 comprises a nucleotide sequence as set forth in SEQ ID NO: 7.

123. The method according to any one of claims 119 to 122, wherein R12 comprises a nucleotide sequence as set forth in SEQ ID NO: 8.

124. The method according to any one of claims 1 to 123, wherein the antigen-binding protein comprises an antibody or an antibody fragment.

125. The method according to claim 124, wherein the antibody fragment comprises scFv, Fab, Fab', (Fab)₂ and/or (Fab')₂.

126. The method according to any one of claims 1 to 125, wherein the directional linkage comprises the use of a ligase.

127. The method according to claim 126, wherein the ligase is a DNA ligase.

128. A method for preparing an antigen-binding protein, comprising expressing the first replacement vector of any one of claims 1 to 127, the second replacement vector of any one of claims 83 to 127, and/or the double-replacement vector of any one of claims 42 to 127, under conditions allowing expression of the first replacement vector of any one of claims 1 to 127, the second replacement vector of any one of claims 83 to 127, and/or the double-replacement vector of any one of claims 42 to 127.

129. A first replacement vector prepared according to the method of any one of claims 1 to 127.

130. A second replacement vector prepared according to the method of any one of claims 83 to 127.

131. A double-replacement vector prepared according to the method of any one of claims 42 to 127.

132. A functional antigen-binding protein prepared by means of the method of any one of claims 1 to 127.
